# EUROPEAN PATENT SPECIFICATION

(11) **EP 0 772 624 B1**
(45) Date of publication and mention of the grant of the patent: **13.09.2000**
(21) Application number: 94915781.2
(22) Date of filing: 06.04.1994
(51) Int. Cl.: C12N 15/24, C12N 1/20, A61K 38/20, C07K 14/54

(54) **INTERLEUKIN-15**
INTERLEUKIN-15
INTERLEUKINE-15

(43) Date of publication of application: 14.05.1997
(73) Proprietor: IMMUNEX CORPORATION, Seattle Washington 98101 (US)
(72) Inventor: GRABSTEIN, Kenneth, H., Mercer Island, WA 98040 (US); ANDERSON, Dirk, M., Seattle, WA 98107 (US); EISENMAN, June, R., Seattle, WA 98119 (US); RAUCH, Charles, Bainbridge Island, WA 98110 (US); FUNG, Victor, Redmond, WA 98053 (US)
(74) Representative: Bassil, Nicholas Charles
(86) International application number: US9403793
(87) International publication number: WO9527722

(56) References cited:
- JOURNAL OF CELLULAR BIOCHEMISTRY, KEYSTONE SYMPOSIA ON MOLECULAR & CELLULAR BIOLOGY, SUPPLEMENT 18D, no. V 561, 26 February 1994 - 17 April 1994, page 400 XP002030602 J.G. GIRI ET AL.: "Biochemical characterization of a novel cytokine IL-15 and its receptor"
- CHALLENGES MOD. MED. , vol. 8, 1994, pages 79-86, XP000670984 D. COSMAN ET AL.: "IL-15, a novel T cell growth factor, that shares components of the receptor for IL-2."
- JOURNAL OF EXPERIMENTAL MEDICINE, vol. 180, no. 4, 1994, pages 1395-1403, XP000670920 W.E. CARSON ET AL.: "Interleukin {IL} 15 is a novel cytokine that activates human natural killer cells via components of the IL-2 receptor."
- CIBA FOUNDATION SYMPOSIUM, vol. 195, 1995, pages 221-229, XP000670929 D. COSMAN ET AL.: "Interleukin 15 and its receptor."
- SCIENCE, Vol. 264, issued 13 May 1994, K.H. GRABSTEIN et al., "Cloning of a T Cell Growth Factor that Interacts with the Beta Chain of the Interleukin-2 Receptor", pages 965-968.

## Description

### FIELD OF THE INVENTION

The present invention relates generally to a mammalian epithelium-derived T-cell factor ("ETF") polypeptide referred to herein as interleukin-15 ("IL-15"). It more particularly relates to an isolated cDNA sequence encoding a polypeptide having IL-15 biological activity, isolated IL-15 polypeptide sequences and derivatives, processes for making IL-15 polypeptides using recombinant DNA technology, a method for inducing T cell proliferation and differentiation, and compositions containing IL-15 providing anti-tumor and anti-infectious disease immunity.

### BACKGROUND OF THE INVENTION

T-cells, also known as T-lymphocytes, are a class of immune effector cells. In peripheral tissues, T -cells can be divided into two broad groups based on their mutually exclusive expression of CD4 and CD8 cell surface molecules. Typical CD8⁺ T-cells become cytotoxic T-cells after activation and destroy antigen bearing target cells through direct cell contact. Activated CD4⁺ T-cells generally provide positive signals, for example, "helper" function for B cells (that enable B cells to differentiate into antibody-forming cells) and, therefore, are called helper T-cells.

Six T-cell growth factors have previously been identified. The six are: Interleukin (IL) -2, -4, -7, -9, -12 and cofactor IL-10. IL-2's open reading frame codes for a 15 kDa, 153-amino acid polypeptide. IL-2 is produced by certain T-cells and by large granular lymphocytes. IL-2 was originally discovered as a factor that would support long-term growth of human T-cells. In addition to T-cell growth, its effects include activation of natural killer (NK) cells and lymphokine-activated killer (LAK) cells as well as cytotoxic T-cells ("CTL"), macrophages and promotion of B-cell growth.

IL-4 is a 15-20 kDa protein produced by activated T-cells, bone marrow stromal cells, and mast cells. The IL-4 open reading frame codes for 140-amino acid murine IL-4 and 153-amino acid human IL-4. Originally, IL-4 was defined as a factor that activated B-cell growth and differentiation. Its effects also include macrophage activation and induction of class II MHC molecules, growth of some T-cell and mast cell lines, proliferation and CTL generation from human peripheral blood T-cells, enhancement of immunoglobulin production by B-cells, and a cofactor in growth of hematopoietic cells from stem cells. IL-4 plays an important role in the down-regulation of IL-2 induced NK cell and LAK cell activities. Human IL-4 is not active on murine cells.

IL-7 is a 20-25 kDa, 177 amino acid polypeptide produced by bone marrow and thymic stromal cells. Although it was originally described as a pit-B-cell growth factor, IL-7 supports the growth of pro-B-cells as well as pre-B-cells. IL-7 also induces proliferation and CTL generation from human peripheral blood T-cells IL-2 receptor expression, IL-2 production, and proliferation in CD4⁺ and CD8⁺ cells. IL-7 also synergizes with IL-2 and increases thymic T-cell proliferation and induces proliferation of CD4⁻ and CD8⁻ thymocytes.

IL-9 is a 30-40 kDa, 144 amino acid polypeptide produced by activated T-lymphocytes. IL-9 was first identified as a helper T-cell growth factor. IL-9 stimulates erythroid development and enhances IL-3 induced proliferation of bone marrow-derived mast cells. It also modulates IgE and IgG production by B-cells in the presence of IL-4. Murine IL-9 is active on human cells, whereas human IL-9 does not act on murine cells.

Human IL-10 is a 16-20 kDa, 178-amino acid polypeptide produced by macrophages and TH2 but not TH1 T-helper cells. Like IL-2, IL-4 and IL-7, IL-10 has several different biological activities. IL-10 was discovered on the basis of its ability to inhibit cytokine production by activated T-cells. Both human and murine IL-10 are growth-stimulatory cofactors for thymocytes and T-cells in combination with IL-7 or IL-2 plus IL-4. IL-10 stimulates mast cell viability and growth in combination with IL-4 or IL-3 plus IL-4. IL-10 also induces the IgG secretion and expression of MHC class II molecules on B-cells and increases their viability in culture.

IL-12 is constitutive or induced by phorbol ester and calcium ionophore in lymphoblastoid cell lines and is produced by LPS stimulated macrophages. IL-12 has a molecular weight of 70 kDa and an unusual hererodimeric structure, being formed of two disulphide-bonded glycoproteins. The larger of the two glycoprotein subunits is a 40 kDa, 328-amino acid polypeptide. The smaller glycoprotein subunit is a 35 kDa, 253-amino acid polypeptide. Both glycoprotein subunits are necessary for bioactivity. IL-12 induces the proliferation of activated T-cells of both the CD4⁺ and CD8⁺ subsets independently of IL-2. IL-12 also activates NK-cell-mediated cytotoxicity and synergizes with IL-2 to generate LAK cells. Unlike IL-2 and IL-7, but similar to IL-4, IL-12 causes little or no proliferation of resting peripheral blood mononuclear cells.

Giri *et al J. Cell. Biol*., Supplement 18D, page 400, Abstract No. V561 from "Keystone Symposia on Molecular and Cellular Biology February 26-April 17, 1994 reported the biochemical characterisation of a novel cytokine, designated IL-15. The abstract did not describe any sequence information relating to this cytokine.

### SUMMARY OF THE INVENTION

A novel T-cell growth factor, hereinafter referred to as "Interleukin-15" ("IL-15"), has been isolated and purified. A cDNA sequence encoding a simian IL-15 polypeptide was isolated that has a 483-bp 5' noncoding region preceding an open reading frame of 489 bp and a 303-bp 3' noncoding region. A cDNA sequence encoding a human IL-15 polypeptide has a 316-bp 5' noncoding region preceding an open reading frame of 489 bp and a 397-bp 3' noncoding region. The nucleotide sequences and deduced amino acid sequences of simian and human open reading frames are disclosed in SEQ ID NOS 1 and 4. Both the simian and human open reading frames encode a precursor polypeptide (SEQ ID NOS 2 and 5). The precursor polypep tides each comprise a 48-amino acid leader sequence and a sequence encoding mature simian or human IL-15 polypeptides. The active simian and human IL-15 polypeptides are disclosed in SEQ ID NO 3 and 6, respectively.

The present invention further comprises other IL-15 polypeptides encoded by nucleotide sequences that hybridize, under moderate to high stringency conditions, to probes defined by nucleotides 145 trough 489 of SEQ ID NOS 1 or 4 or to their complementary DNA or RNA strands, and that code on expression for polypeptides that stimulate T-lymphocytes to proliferate and differentiate. The invention further comprises nucleotide sequences that, due to the degeneracy of the genetic code, encode IL-15 polypeptides encoded by the nucleotide sequences described above and sequences complementary to them.

Further still, the invention provides for recombinant DNA molecules comprising the foregoing nucleotide sequences, for example, expression vectors or plasmids and transformed host cells, that are useful in producing IL-15 polypeptides, and processes for producing recombinant IL-15 polypeptides using such molecules.

### BRIEF DESCRIPTION OF THE DRAWINGS

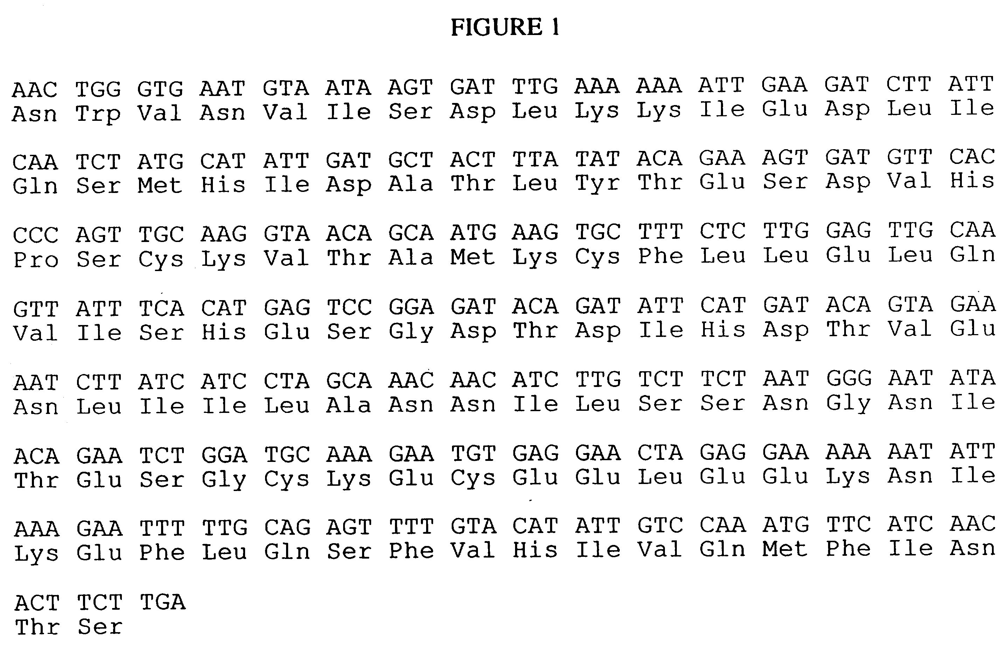

Figure 1 shows the nucleotide sequence and deduced amino acid sequence of the active simian species of IL-15.

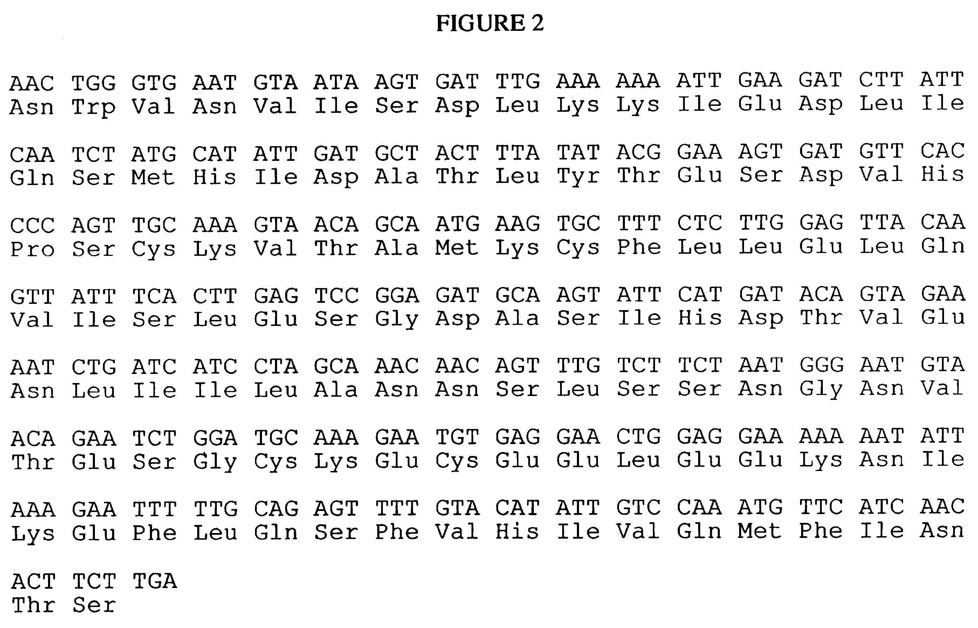

Figure 2 shows the nucleotide sequence and deduced amino acid sequence of the active human species of IL-15.

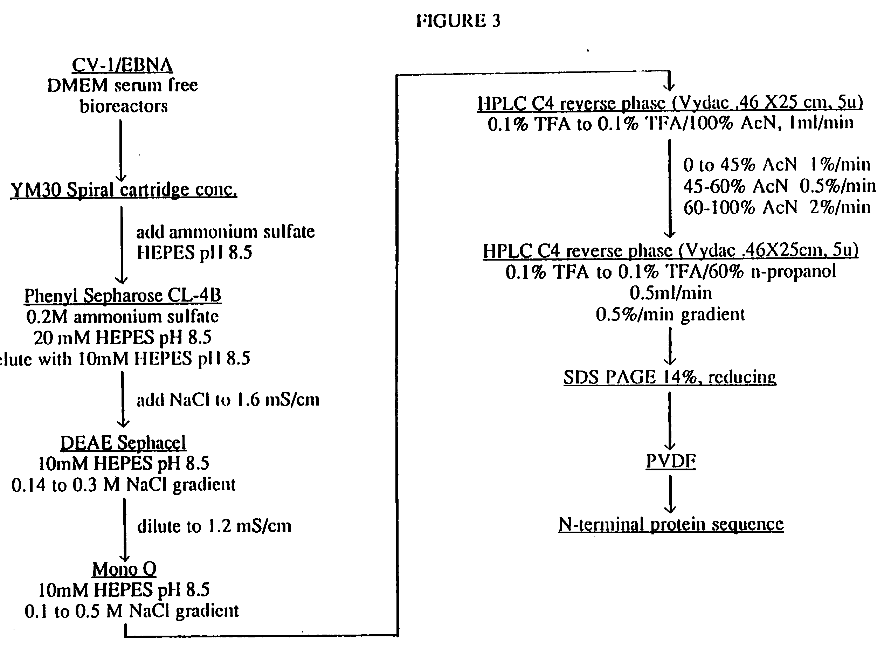

Figure 3 shows a purification and protein sequencing scheme useful in isolating IL-15 polypeptides.

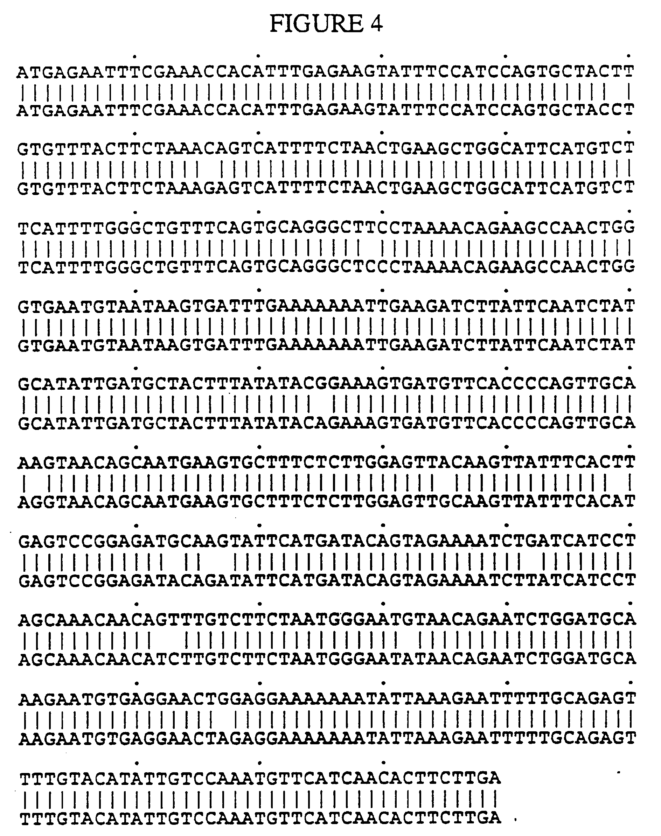

Figure 4 shows the homology between nucleotide sequences encoding human and simian species of IL-15. The human sequence is shown above the simian sequence.

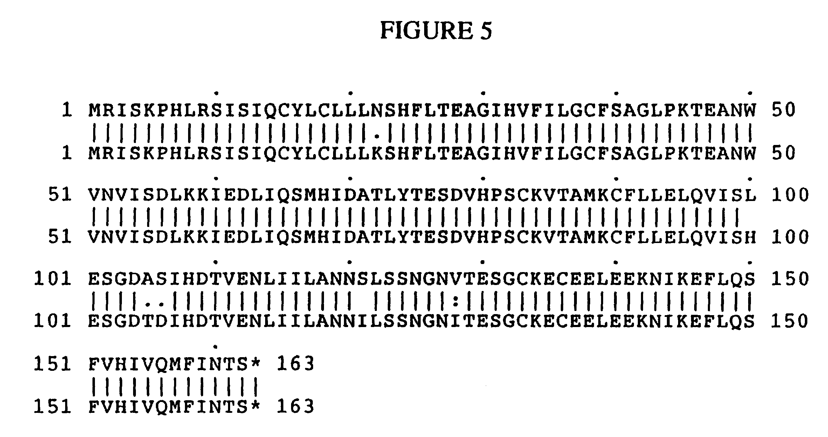

Figure 5 shows the homology between amino acid sequences of human and simian species of IL-15. The human sequence is shown above the simian sequence. In both species, the leader sequence (amino acids 1 through 48) is cleaved from the precursor polypeptide to form the mature polypeptide (amino acids 49 through 162).

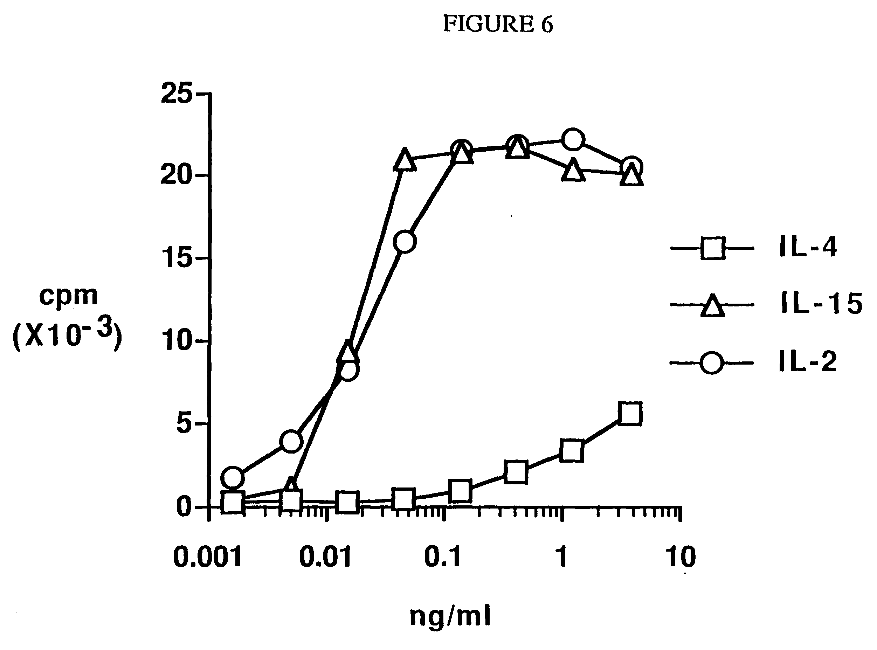

Figure 6 shows the biological activity recombinant IL-15, IL-2 and IL-4 *in vitro* using CTLL-2 cells. The *in vitro* proliferative response of CTLL-2 cells was measured at increasing concentrations of recombinant cytokine (expressed as ng/ml). The data are expressed as cpm of ³H-thymidine incorporated (X 10⁻³).

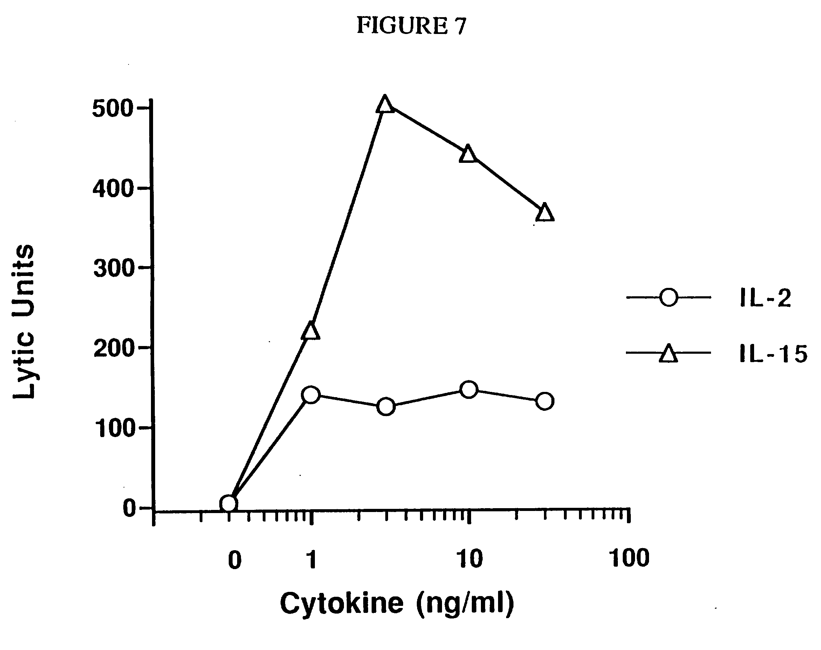

Figure 7 shows the induction of CTL lytic activity by rIL- 15 and IL-2. Antigen specific cytolytic T lymphocytes (CTL) were generated *in vitro*. Human peripheral blood mononuclear cells (PBL) from one donor were stimulated with irradiated PBL from an allogeneic donor in cultures containing various concentrations of either IL-2 or human rIL-15. Cultures were assayed for cytolytic activity against ⁵¹Cr labeled targets derived from the original stimulating donor. Lytic units were calculated as the inverse of the fraction of the responding culture required to generate 50% of the maximum specific ⁵¹Cr release.

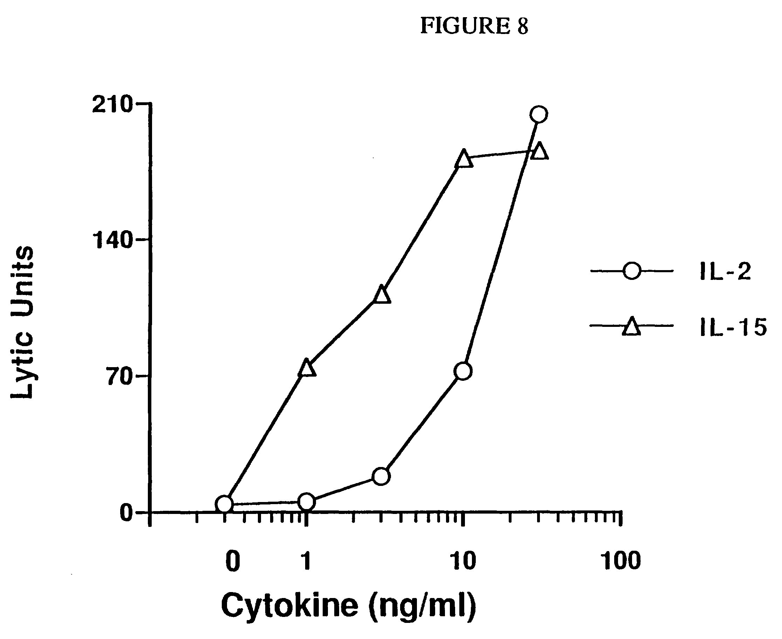

Figure 8 shows the induction of LAK cells lytic activity by rIL-15 and IL-2. Lymphokine activated killer (LAK) cells were generated *in vitro*. Human PBL were stimulated with irradiated autologous PBL and cytolytic activity was measured against the Daudi lymphoblastoid cell line. Lytic units were calculated as the inverse of the fraction of the responding culture required to generate 30% of the maximum specific ⁵¹Cr release.

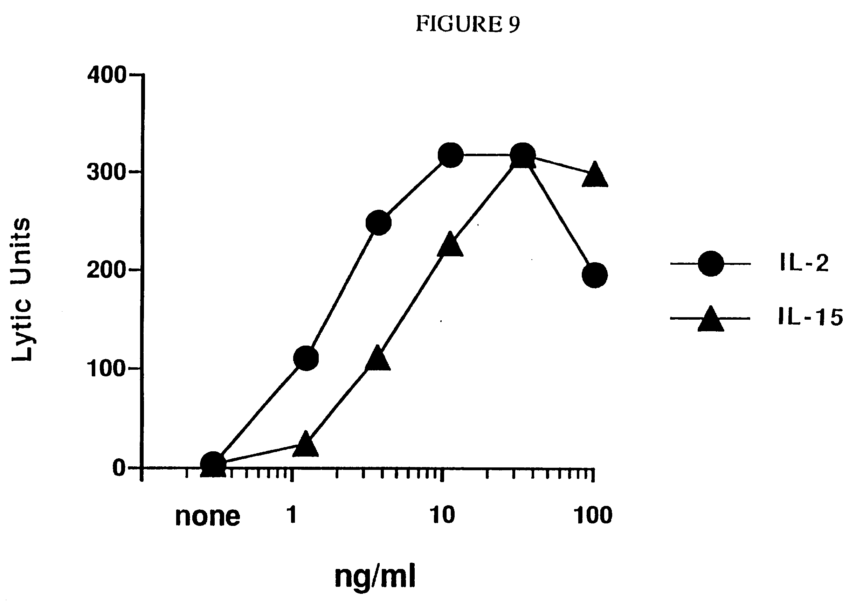

Figure 9 shows the induction of NK cell lytic activity by rIL-15 and IL-2. Natural killer (NK) cells were isolated from whole human PBL isolated by antibody affinity to paramagnetic microspheres using monoclonal antibodies against CD16. The purified NK cells were cultured for 3 days and cytolytic activity was measured against the K562 erythroleukemia cell line.

### DETAILED DESCRIPTION OF THE INVENTION

"Interleukin-15" or "IL-15" refers to mammalian polypeptides that are structurally similar to the polypeptides disclosed herein and that stimulate T-lymphocytes to proliferate and differentiate. IL-15 is distinguishable from IL-2, IL-4, IL-7, IL-9, IL-10, and IL-12 in structure and cellular origin (Table 1). In primates, IL-15 polypeptide is initially produced by epithelial cells as a 162-amino acid precursor IL-15 polypeptide. This precursor contains a 48-amino acid leader sequence that is removed from the precursor polypeptide to form the mature polypeptide. The mature IL-15 polypeptide is capable of signaling proliferation and/or differentiation of precursor or mature T-cells. The protein, therefore, can be used to promote long-ter*m in vitro* culture of T-lymphocytes and T-cell lines.

"sIL-15" refers to a simian species of IL-15. "hIL-15" refers to a human species of IL-15. "rIL-15" refers to recombinant IL-15. Both purified sIL-15 and rIL-15 will stimulate proliferation of CTLL-2 cells (Gillis and Smith, *Nature* 268:154 (1977); ATCC TIB 214). In the CTLL-2 proliferation assays, supernatants of cells transfected with recombinantly expressed precursor and inframe fusions of mature forms of sIL-15 induced CTLL-2 cell proliferation. For other assays, peripheral blood T-cells ("PBT") and peripheral blood mononuclear leukocytes ("PBL") were isolated from human peripheral blood. We found that rIL-15 stimulated proliferation of PBT and PBL previously cultured with phytohemagglutinin ("PHA"). rIL-15 also stimulated proliferation of PHA activated CD4⁺, and CD8⁺ cells. rIL-15 stimulated proliferation of resting human T-cells or resting murine T-cell clones in the presence of anti-CD3 (T-cell receptor) antibodies. Experiments with PHA activated PBT demonstrate that rIL-15 exerts its growth stimulatory effects independently of IL-2, in that antibodies to IL-2 or to the IL-2 receptor do not inhibit IL-15.

The terms IL-15, sIL-15 and hIL-15 include analogs or subunits of native mammalian polypeptides that are encoded by nucleic acids that bind to the nucleic acid sequences in Figures 1 and 2 (nucleotides 145 through 489, inclusive, in SEQ ID NOS 1 and 4) under conditions of specified stringency and that induce proliferation and differentiation of T-lymphocytes, and stimulate proliferation of T-cell lines and isolated PBT.

"Recombinant DNA technology" or "recombinant", as used herein, refers to techniques and processes for producing specific polypeptides from microbial (e.g., bacterial, fungal or yeast) or mammalian cells or organisms (e.g., transgenics) that have been transformed or

**Table 1**

| **Origin and structure of T-cell growth factors** | | | |
|---|---|---|---|
| FACTOR | SIZE (kDA) | NO. OF AMINO ACIDS* | SOURCE |
| IL-2 | 15 | 153 | Activated lymphocytes |
| IL-4 | 15-20 | 153 | Activated T-lymphocytes Bone marrow stromal cells Mast cells |
| IL-7 | 25 | 177 | Bone marrow and thymic stromal cells |
| IL-9 | 30-40 | 144 | Activated T-lymphocytes |
| IL-10 | 16-20 | 178 | Th2 lymphocytes |
| Il-12** | 40 and 35 | 328 and 253 | B-lymphoblastoid cell lines |
| IL-15 | 15-17 | 162 | Epithelial cells |

| | | | |
|---|---|---|---|
| * Number in human polypeptides encoded by the open reading frame. | | | |
| ** Two glycoprotein subunits are necessary for activity. | | | |

transfected with cloned or synthetic DNA sequences to enable biosynthesis of heterologous peptides. Native glycosylation patterns will only be achieved with mammalian cell expression systems. Yeast provide a distinctive glycosylation pattern. Prokaryotic cell expression (e.g., *E. coli)* will generally produce polypeptides without glycosylation.

"Biologically active" means that a particular IL-15 polypeptide is capable of stimulating T-lymphocyte proliferation and/or differentiation. In the case of sIL-15 and hIL-15, this biological activity also corresponds to stimulation of the proliferation of murine or primate, for example, human, T-cell lines or PBT.

A "nucleotide sequence" refers to a polynucleotide in the form of a separate fragment or as a component of a larger DNA construct, that has been derived from DNA isolated at least once in substantially pure form (i.e., free of contaminating endogenous materials) and in a quantity or concentration enabling identification, manipulation, and recovery of its component nucleotide sequences by standard biochemical methods (such as those outlined in Sambrook et al., Molecular Cloning: A Laboratory Manual, 2nd ed., Cold Spring Harbor Laboratory, Cold Spring Harbor, NY (1989)) such as a cloning vector. Such sequences are preferably provided in the form of an open reading frame uninterrupted by internal nontranslated sequences, or introns, that are typically present in eukaryotic genes. Sequences of non-translated DNA may be present 5' or 3' from an open reading frame, where the same do not interfere with manipulation or expression of the coding regions.

"Recombinant expression vector" refers to a plasmid comprising a transcriptional unit comprising an assembly of (1) a genetic element or elements having a regulatory role in gene expression, for example, promoters or enhancers, (2) a structural or coding sequence that is transcribed into mRNA and translated into a polypeptide having IL-15 biological activity, and (3) appropriate transcription and translation initiation and termination sequences. The various regulatory elements that can be used are discussed below (see Recombinant DNA Techniques). Structural elements intended for use in yeast expression systems preferably include a leader sequence enabling extracellular secretion of translated polypeptide by a yeast host cell. Alternatively, in a bacterial expression system, the recombinant polypeptide may include a N-terminal methionine residue. The N-terminal methionine residue may be subsequently cleaved from the expressed recombinant polypeptide to provide a product suitable for further purification.

"Recombinant microbial expression system" refers to a substantially homogeneous monoculture of suitable host microorganisms, for example, bacteria, such as *E. coli*, or yeast, such as *S. cerevisiae*, that have stably integrated a recombinant transcriptional unit into chromosomal DNA or carry the recombinant transcriptional unit as a component of a resident plasmid. Generally, host cells constituting a recombinant microbial expression system are the progeny of a single ancestral transformed cell. Recombinant microbial expression systems will express heterologous polypeptides upon induction of the regulatory elements linked to a structural nucleotide sequence to be expressed.

Transformed host cells are cells that have been transformed or transfected with a recombinant expression vector. Expressed mammalian IL-15 will be located within the host cell and/or secreted into culture supernatant, depending upon the nature of the host cell and the gene construct inserted into the host cell.

Moderate stringency hybridization conditions, as defined herein and as known to those skilled in the art, refer to conditions described in, for example, Sambrook et al., *supra*, Vol. 2, pp. 8.46-8.49 and 9.47-9.55. Conditions of moderate stringency, as defined by Sambrook et al. include, for example, overnight hybridization and post-hybridization washes at 55°C, 5 x SSC, 0.5% SDS. Severe or high stringency conditions include higher temperatures of hybridization and post-hybridization washes, or lower salt concentrations.

### IL-15 Polypeptides

We have purified a simian species of IL-15 (sIL-15) and sequenced the N-terminal peptide of mature sIL-15. Using the N-terminal amino acid sequence and PCR, we isolated a cDNA encoding sIL-15 and determined the nucleotide sequence and deduced amino acid sequence of mature sIL-15 (Figure 1), and the nucleotide sequence and deduced amino acid sequence of a precursor of sIL-15 polypeptide (SEQ ID NO 1 and SEQ ID NO 2). Precursor IL-15 polypeptide sequence in the simian species comprises a mature active protein (SEQ ID NO 3) preceded by a 48-amino acid leader sequence. The leader sequence isamino acids 1-48 of SEQ ID NO 2. Primate IL-15 stimulates proliferation of murine T-cell lines (e.g., CTLL-2) and stimulates proliferation and differentiation of human PBT cells.

The present invention also comprises other mammalian IL-15, including human IL-15, having IL-15 biological activity and encoded by nucleotide sequences that hybridize, under conditions of moderate to high stringency, to probes defined by SEQ ID NO 5. A plasmid containing a recombinant clone of human IL-15 cDNA was deposited with the American Type Culture Collection, 12301 Parklawn Drive, Rockville, MD 20852 USA ("ATCC") in accordance with the Budapest Treaty on the International Recognition of the Deposit of Microorganisms for the Purposes of Patent Procedure on February 19, 1993 under accession number ATCC 69245. The deposit was named "I41-hETF" and comprised an *E. coli* strain containing plasmid hETF/pDC406 that contained a 316-bp 5' noncoding region preceding an open reading frame of 489 bp and a 397-bp 3' noncoding region flanked by the *Sal* I adaptors shown in SEQ ID NOS 7 and 8. All restrictions on the availability to the public of the material deposited will be irrevocably removed upon the granting of a patent.

The amino acid structure of IL-15 polypeptides disclosed herein may be modified by forming covalent or aggregative conjugates with other chemical moieties, such as glycosyl groups, lipids, phosphate, acetyl groups and the like, or by creating amino acid sequence mutants. Covalent derivatives of mammalian IL-15 are prepared by linking particular functional groups to mammalian IL-15 amino acid side chains or at the N-terminus or C-terminus of a mammalian IL-15 polypeptide. Other derivatives of mammalian IL-15 within the scope of this invention include covalent or aggregative conjugates of mammalian IL-15 or its fragments with other proteins or polypeptides, such as by synthesis in recombinant culture as N-terminal or C-terminal fusions. For example, the conjugated polypeptide may be a signal (or leader) polypeptide sequence at the N-terminal region of a mammalian IL-15 polypeptide for transport from its site of synthesis to a site inside or outside of the cell membrane or wall (e.g., the yeast α-factor leader). Further, using conventional techniques, IL-15 polypeptides can be expressed as polypeptide fusions comprising additional polypeptide sequences, such as Fc or other immunoglobulin sequences, linker sequences, or other sequences that facilitate purification and identification of IL-15 polypeptides. Still further, IL-15 polypeptide fusions can comprise fusions with other cytokines to provide novel polyfunctional entities. Other cytokines include, for example, any of interleukins-1 through 13, tumor necrosis factor (TNF), granulocyte macrophage-colony stimulating factor (GM-CSF), granulocyte-colony stimulating factor (G-CSF), mast cell growth factor (MGF) and other cytokines that affect immune cell growth, differentiation or function.

The present invention further includes IL-15 polypeptides having altered glycosylation. IL-15 polypeptides expressed in yeast or mammalian expression systems (e.g., COS-7 cells (ATCC CRL 1651)) may be similar or significantly different in molecular weight and glycosylation pattern than a native IL-15 polypeptide. This depends upon the choice of expression system. Expression of IL-15 polypeptides in bacterial expression systems, such as *E. coli*, provide non-glycosylated molecules.

Functional mutant analogs of human or other mammalian IL-15 can be synthesized, for example, with inactivated N-glycosylation sites by oligonucleotide synthesis and ligation or by site-specific mutagenesis techniques. IL-15 polypeptide derivatives can be expressed in homogeneous, reduced carbohydrate form using yeast expression systems. N-glycosylation sites in eukaryotic polypeptides are characterized by an amino acid triplet Asn-Φ-Ω where Φ is any amino acid except Pro and Ω is Ser or Thr. An IL-15 mutant derivative, as referred to herein, is a polypeptide substantially homologous to a sequence of a native mammalian IL-15 but that has an amino add sequence different from a native mammalian IL-15 polypeptide because of a deletion, insertion or substitution.

Bioequivalent analogs of IL-15 polypeptides of IL-15 muteins may be constructed by making various substitutions of amino acid residues or sequences, or by deleting terminal or internal residues or sequences not needed for biological activity. For example, Cys residues can be deleted or replaced with other amino acids to prevent formation of incorrect intramolecular disulfide bridges upon renaturation. Other approaches to mutagenesis involve modification of dibasic amino acid residues to enhance expression in yeast systems in which KEX2 protease activity is present. Generally, substitutions are made conservatively by substituting an amino acid having physiochemical characteristics resembling those of the native residue.

Antisense or sense oligonucleotides comprise single-stranded nucleic acid sequences (either RNA or DNA) capable of binding to sense IL-15 mRNA or antisense IL-15 cDNA sequences. An antisense or sense oligonucleotide, according to the present invention, comprises a fragment of the nucleotide sequences in Figures 1 or 2 or a DNA or RNA complement of the nucleotide sequences in Figures 1 and 2. Such a fragment comprises at least about 14 nucleotides and is capable of binding to IL-15 DNA. The ability to create an antisense or a sense oligonucleotide, based upon a cDNA sequence for IL-15 is described in, for example, Stein and Cohen, *Cancer Res*. 48:2659 (1988), and van der Krol et al., *BioTechniques* 6:958 (1988).

Isolation and characterization of IL-15 from non-recombinant cellular sources requires a mammalian cell line that produces IL-15 and a responder cell line that proliferates in response to IL-15 stimulation. A biological assay for mammalian IL-15 may employ a growth factor-dependent T-cell line as a detector of factors that induce lymphoid cell proliferation. T-cells isolated from blood samples taken from humans or from other mammals also can be used to assay mammalian IL-15 polypeptides.

An IL-15-dependent cell line can be derived from murine CTLL-2 cells. This cell line responds to purified human, murine, and recombinant IL-2 and murine IL-4 but not to IL-1, IL-3, human IL-4, or any of the other known growth factors.

One can utilize the simian or human IL-15 cDNA sequences disclosed herein to obtain cDNAs encoding other mammalian homologs of simian or human IL-15 by cross-species hybridization techniques. Briefly, an oligonucleotide probe is created from the nucleotide sequence of the protein coding region of sIL-15 cDNA as described in Figure 1 or SEQ ID NO 1 or hIL-15 cDNA as described in Figure 2 or SEQ ID NO 4. This probe can be made by standard techniques, such as those described in Sambrook et al. *supra*. The simian or human probe is used to screen a mammalian cDNA library or genomic DNA library under moderate stringency conditions. Mammalian cDNA libraries can be made from mRNAs isolated from, for example, murine peripheral blood lymphocytes. Alternatively, other cDNA libraries or mRNAs isolated from various tissues or cell lines can be screened by Northern hybridization to determine a suitable source of mammalian IL-15 DNA or mRNA.

### CV-1/EBNA IL-15 Purification

We have purified IL-15 to provide an isolated polypeptide preparation from a non-homogeneous protein solution, such as conditioned medium collected from cells expressing IL-15. IL-15 activity in crude conditioned medium samples is not always detectable using currently available IL-15 bioassay techniques. At least one purification step is typically required before IL-15 biological activity is detectable utilizing bioassay techniques described herein.

A non-homogeneous protein solution, e.g., conditioned medium, is prepared by growing the CV-1/EBNA line (C. J. McMahan et al., *EMBO J.*, 10(10):2821-2832 (1991); ATCC CRL 10478) of African Green Monkey kidney cells in a tissue culture medium. Preferably, the medium is high glucose Dulbecco's Modified Essential Medium ("DMEM", Gibco). Most preferably, a growth medium and a production medium are used. The growth medium employed in isolating sIL-15 as described herein was high glucose (4500 mg/L) DMEM supplemented with 7.5% fetal bovine serum, 50 u/ml penicillin, 50 ug/ml streptomycin, 3 to 4.0 mM L-glutamine, 1 mM sodium pyruvate, 0.1 mM non-essential amino acids and 10 mM N-[2-hydroxyethyl]piperazine-N'-[2-ethanesulfonic acid] ("HEPES") buffer. A serum free production medium of DMEM without phenol red supplemented with 50 u/ml penicillin, 50 ug/ml streptomycin, 3 to 4.0 mM L-glutamine, 1 mM sodium pyruvate, 0.1 mM non-essential amino acids and 10 mM HEPES buffer was developed for IL-15 production and purification. The CV-1/EBNA cells were attachment dependent and could be grown in dishes, flasks, roller bottles or microcarriers.

More specifically, IL-15 was produced by culturing CV-1/EBNA cells on microcarriers in a controlled bioreactor. Cell stocks were maintained in roller bottle flasks. To start a production cycle, cells were trypsinized and inoculated into a spinner flask containing the growth medium described above and 5 g/l Cytodex® 3 microcarriers (Pharmacia). Initial seeding density ranged from 1.5 to 3.5 x 10⁵ cells /ml. To ensure efficient cell attachment to the microcarriers, the cells were kept in the spinner flask for 2 to 24 hours. Spinner flask cultures were incubated at 37°C and agitated at 25 to 40 RPM. After the attachment period, the culture was transferred to a controlled bioreactor. Bioreactor temperature, pH, oxygen and agitation set points were 37°C, 7.0, 20% saturation (relative to air), and 75-85 RPM, respectively. For routine observation of cell growth and health, samples were observed by bright field microscopy. Quantification of cell growth was achieved by counting released nuclei after treatment with a solution of 100 mM citric acid and 0.1% crystal violet.

The culture was supplemented with additional growth medium when ammonia levels reached 5.0 mM. This was repeated until the microcarriers were confluent. The culture medium was then exchanged to the serum-free production medium described above. This procedure was accomplished by allowing the microcarriers to settle to the bottom of the reactor, aspirating the growth medium and replacing it with production medium. This was repeated until an approximately 3,125 fold dilution was achieved. Two to six rounds of production can be expected. In each round, cells were allowed to produce for four to seven days after which 80% of the production medium was collected. This was repeated until the cells were completely detached from the microcarriers.

Approximately 64 liters of CV-1/EBNA conditioned media were used to purify and provide protein for the N- amino acid sequence of sIL-15. As shown in Figure 3, the purification scheme included ultrafiltration, hydrophobic chromatography, anion exchange chromatography, reverse phase high performance liquid chromatography (RP-HPLC), and sodium dodecyl sulfate polyacrylamide gel electrophoresis (SDS-PAGE). Protein was sequenced by blotting the SDS gel to a polyvinylidene fluoride ("PVDF") membrane and determining an N-terminal amino acid sequence by Edman degradation directly from the PVDF membrane. N-terminal amino acid sequencing revealed the first 33 amino acids shown in SEQ ID NO 3. Subsequent sequencing of a cDNA clone obtained from a CV-1/EBNA cDNA library produced a DNA sequence encoding the polypeptide of SEQ ID NO 2. This clone includes a relatively short 48 amino acid leader sequence from SEQ ID NO 2 and a mature polypeptide represented by SEQ ID NO 3.

### Recombinant DNA Techniques

Human, simian and other mammalian IL-15 polypeptides are preferably produced by recombinant DNA techniques. Such techniques involve insertion of a cDNA encoding a human or other mammalian IL-15 polypeptide or a derivative thereof into an expression vector.

Recombinant production of mammalian IL-15 polypeptides or derivatives thereof first requires isolation of a DNA clone (i.e., cDNA) that codes on expression for a mammalian IL-15 polypeptide or a derivative thereof. cDNA clones are derived from primary cells or cell lines that express mammalian IL-15 polypeptides. First total cell mRNA is isolated, then a cDNA library is made from the mRNA by reverse transcription. A cDNA clone may be isolated and identified using the DNA sequence information provided herein to design a cross-species hybridization probe or PCR primer as described above.

The isolated cDNA is preferably in the form of an open reading frame uninterrupted by internal nontranslated sequences, or introns. Genomic DNA containing the relevant nucleotide sequences that code for expression of mammalian IL-15 polypeptides can also be used as a source of genetic information useful in constructing coding sequences. The isolated cDNA can be mutated by techniques known in the art to promote IL-15 derivatives or analogs that exhibit IL-15 biological activity.

Recombinant expression vectors include synthetic or cDNA-derived DNA fragments encoding IL-15 or biologically active derivatives thereof. The DNA encoding a IL-15 or a derivative thereof is operably linked to a suitable transcriptional or translational regulatory or structural nucleotide sequence, such as one derived from mammalian, microbial, viral or insect genes. Examples of regulatory sequences include, for example, a genetic sequence having a regulatory role in gene expression (e.g., transcriptional promoters or enhancers), an optional operator sequence to control transcription, a sequence encoding suitable mRNA ribosomal binding sites, and appropriate sequences that control transcription and translation initiation and termination. Nucleotide sequences are operably linked when the regulatory sequence functionally relates to the structural gene. For example, a DNA sequence for a signal peptide (secretory leader) may be operably linked to a structural gene DNA sequence for a mammalian IL-15 or derivative thereof if the signal peptide is expressed as part of a precursor amino acid sequence and participates in the secretion of a mammalian IL-15. Further, a promoter nucleotide sequence is operably linked to a coding sequence (e.g., structural gene DNA) if the promoter nucleotide sequence controls the transcription of the structural gene nucleotide sequence. Still further, a ribosome binding site may be operably linked to a structural gene nucleotide coding sequence (e.g., mammalian IL-15) if the ribosome binding site is positioned within the vector to encourage translation.

Suitable host cells for expression of mammalian IL-15 or derivatives thereof include prokaryotes, yeast or higher eukaryotic cells under the control of appropriate promoters. Prokaryotes include gram negative or gram positive organisms, for example *E. coli* or bacilli. Suitable prokaryotic hosts cells for transformation include, for example, *E. coli, Bacillus subtilis, Salmonella typhimurium,* and various other species within the genera *Pseudomonas*, *Streptomyces*, and *Staphylococcus*. As discussed in greater detail below, examples of suitable host cells also include yeast such as *S. cerevisiae*, a mammalian cell line such as Chinese Hamster Ovary (CHO) cells, or insect cells. Cell-free translation systems could also be employed to produce mammalian IL-15 or derivatives thereof using RNAs derived from the DNA constructs disclosed herein. Appropriate cloning and expression vectors for use with bacterial, fungal, yeast, and mammalian cellular hosts are described, for example, in Pouwels et al. Cloning Vectors: A Laboratory Manual, Elsevier, New York, 1985.

When a mammalian IL-15 or derivative thereof is expressed in a yeast host cell, the nucleotide sequence (e.g., structural gene) that codes on expression for a mammalian IL-15 or derivative thereof may include a leader sequence. The leader sequence may enable improved extracellular secretion of translated polypeptide by a yeast host cell.

Mammalian IL-15 may be expressed in yeast host cells, preferably from the *Saccharomyces* genus (e.g., *S. cerevisiae*). Other genera of yeast, such as *Pichia* or *Kluyveromyces*, may also be employed. Yeast vectors will often contain an origin of replication sequence from a 2µ yeast plasmid, an autonomously replicating sequence (ARS), a promoter region, sequences for polyadenylation, and sequences for transcription termination. Preferably, yeast vectors include an origin of replication sequence and selectable marker. Suitable promoter sequences for yeast vectors include promoters for metallothionein, 3-phosphoglycerate kinase (Hitzeman et al., *J. Biol. Chem. 255*:2073, 1980) or other glycolytic enzymes (Hess et al., *J. Adv. Enzyme Reg. 7*:149, 1968; and Holland et al., *Biochem*. *17*:4900, 1978), such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase. Other suitable vectors and promoters for use in yeast expression are further described in Hitzeman, EP-A-73,657.

Yeast vectors can be assembled, for example, using DNA sequences from pBR322 for selection and replication in *E. coli* (Amp^{r} gene and origin of replication). Other yeast DNA sequences that can be included in a yeast expression construct include a glucose-repressible ADH2 promoter and α-factor secretion leader. The ADH2 promoter has been described by Russell et al. (*J. Biol. Chem. 258*:2674, 1982) and Beier et al. (*Nature 300*:724, 1982). The yeast α-factor leader sequence directs secretion of heterologous polypeptides. The α-factor leader sequence is often inserted between the promoter sequence and the structural gene sequence. *See, e.g*., Kurjan et al., *Cell 30*:933, 1982; and Bitter et al, *Proc. Natl. Acad. Sci. USA 81*:5330, 1984. A leader sequence may be modified near its 3' end to contain one or more restriction sites. This will facilitate fusion of the leader sequence to the structural gene.

Yeast transformation protocols are known to those skilled in the art. One such protocol is described by Hinnen er al., *Proc. Natl. Acad. Sci. USA 75*:1929, 1978. The Hinnen et al. protocol selects for Trp⁺ transformants in a selective medium, wherein the selective medium consists of 0.67% yeast nitrogen base, 0.5% casamino acids, 2% glucose, 10 mg/ml adenine and 20 mg/ml uracil.

Yeast host cells transformed by vectors containing ADH2 promoter sequence may be grown for inducing expression in a "rich" medium. An example of a rich medium is one consisting of 1% yeast extract, 2% peptone, and 1% glucose supplemented with 80 mg/ml adenine and 80 mg/ml uracil. Derepression of the ADH2 promoter occurs when glucose is exhausted from the medium.

Alternatively, in a prokaryotic host cell, such as *E. coli*, the mammalian IL-15 or derivative thereof may include an N-terminal methionine residue to facilitate expression of the recombinant polypeptide in a prokaryotic host cell. The N-terminal Met may be cleaved from the expressed recombinant mammalian IL-15.

The recombinant expression vectors carrying the recombinant mammalian IL-15 structural gene nucleotide sequence or derivative thereof are transfected or transformed into a suitable host microorganism or mammalian cell line.

Expression vectors transfected into prokaryotic host cells generally comprise one or more phenotypic selectable markers. A phenotypic selectable marker is, for example, a gene encoding proteins that confer antibiotic resistance or that supply an autotrophic requirement, and an origin of replication recognized by the host to ensure amplification within the host. Other useful expression vectors for prokaryotic host cells include a selectable marker of bacterial origin derived from commercially available plasmids. This selectable marker can comprise genetic elements of the cloning vector pBR322 (ATCC 37017). pBR322 contains genes for ampicillin and tetracycline resistance and thus provides simple means for identifying transformed cells. The pBR322 "backbone" sections are combined with an appropriate promoter and a mammalian IL-15 structural gene sequence. Other commercially available vectors include, for example, pKK223-3 (Pharmacia Fine Chemicals, Uppsala, Sweden) and pGEM1 (Promega Biotec, Madison, WI, USA).

Promoter sequences are commonly used for recombinant prokaryotic host cell expression vectors. Common promoter sequences include β-lactamase (penicillinase), lactose promoter system (Chang et al., *Nature 275*:615, 1978; and Goeddel et al., *Nature 281*:544, 1979), tryptophan (trp) promoter system (Goeddel et al., *Nucl. Acids Res. 8*:4057, 1980; and EPA 36,776) and tac promoter (Sambrook et al., Molecular Cloning: A Laboratory Manual, Cold Spring Harbor Laboratory, (1989)). A particularly useful prokaryotic host cell expression system employs a phage λ P_{L} promoter and a cI857ts thermolabile repressor sequence. Plasmid vectors available from the American Type Culture Collection that incorporate derivatives of the λ P_{L} promoter include plasmid pHUB2 (resident in *E. coli* strain JMB9 (ATCC 37092)) and pPLc28 (resident in *E. coli* RR1 (ATCC 53082)).

Mammalian or insect host cell culture systems also could be employed to express recombinant mammalian IL-15 polypeptide or derivatives thereof. Examples of suitable mammalian host cell lines include the COS-7 lines of monkey kidney cells (Gluzman et al., *Cell 23*:175, (1981); ATCC CRL 1651), L cells, C127 cells, 3T3 cells (ATCC CCL 163), CHO cells, HeLa cells (ATCC CCL 2), and BHK (ATCC CRL 10) cell lines. Suitable mammalian expression vectors include nontranscribed elements such as an origin of replication, a promoter sequence, an enhancer linked to the structural gene, other 5' or 3' flanking nontranscribed sequences, such as ribosome binding sites, a polyadenylation site, splice donor and acceptor sites, and transcriptional termination sequences.

Transcriptional and translational control sequences in mammalian host cell expression vectors may be provided by viral sources. For example, commonly used mammalian cell promoter sequences and enhancer sequences are derived from Polyoma, Adenovirus 2, Simian Virus 40 (SV40), and human cytomegalovirus. DNA sequences derived from the SV40 viral genome, for example, SV40 origin, early and late promoter, enhancer, splice, and polyadenylation sites may be used to provide the other genetic elements required for expression of a structural gene sequence in a mammalian host cell. Viral early and late promoters are particularly useful because both are easily obtained from a viral genome as a fragment that may also contain a viral origin of replication (Fiers et al., *Nature 273*:113, 1978). Smaller or larger SV40 fragments may also be used, provided the approximately 250 bp sequence extending from the *Hind* III site toward the *Bgl* I site located in the SV40 viral origin of replication site is included.

Exemplary mammalian expression vectors can be constructed as disclosed by Okayama and Berg (*Mol. Cell. Biol. 3*:280, 1983). Additional useful mammalian expression vectors are described in U.S. Patent Application Serial No. 07/480,694 filed February 14, 1990 and U.S. Patent Application Serial No. 07/543,193 filed June 5, 1990.

### Purification of Recombinant Mammalian IL-15

IL-15 polypeptides may be prepared by culturing transformed host cells under culture conditions necessary to express mammalian IL-15 polypeptides or derivatives thereof. The resulting expressed polypeptides may then be purified from culture media or cell extracts. A mammalian IL-15 polypeptide or derivative thereof may be concentrated using a commercially available protein concentration filter, for example, an Amicon or Millipore Pellicon ultrafiltration unit. With or without the concentration step, the culture media can be applied to a purification matrix such as a hydrophobic chromatography medium. Phenyl Sepharose® CL-4B (Pharmacia) is the preferred medium. Alternatively, an anion exchange resin can be employed, for example, a matrix or substrate having pendant diethylaminoethyl (DEAE) groups. The matrices can be acrylamide, agarose, dextran, cellulose or other types commonly employed in protein purification. Alternatively, gel filtration medium can be used. Recombinant IL-15 is stable in acidic aqueous buffers and a cation exchange resin also can be used.

Finally, one or more reverse-phase high performance liquid chromatography (RP-HPLC) steps employing hydrophobic RP-HPLC media, e.g., silica gel having pendant methyl or other aliphatic groups, can be employed to further purify IL-15. Some or all of the foregoing purification steps, in various combinations, can also be employed to provide a substantially homogeneous recombinant protein. Alternatively, some or all of the steps used in the purification procedure described above for simian IL-15 can also be employed.

Recombinant protein produced in bacterial culture is usually isolated by initial disruption of the host cells, centrifugation, extraction from cell pellets if an insoluble polypeptide, or from the supernatant if a soluble polypeptide, followed by one or more concentration, salting-out, ion exchange or size exclusion chromatography steps. Finally, RP-HPLC can be employed for final purification steps. Microbial cells can be disrupted by any convenient method, including freeze-thaw cycling, sonication, mechanical disruption, or use of cell lysing agents.

Transformed yeast host cells are preferably employed to express IL-15 as a secreted polypeptide. This simplifies purification. Secreted recombinant polypeptide from a yeast host cell fermentation can be purified by methods analogous to those disclosed by Urdal et al. (*J*. *Chromatog. 296*: 171, 1984). Urdal et al. describe two sequential, reversed-phase HPLC steps for purification of recombinant human IL-2 on a preparative HPLC column.

### Administration of Mammalian IL-15 Polypeptide and Derivative Compositions

The present invention provides methods of using therapeutic compositions comprising an effective amount of IL-15 in a suitable diluent or carder. For therapeutic use, purified IL-15 or a biologically active derivative thereof is administered to a patient, preferably a human, for treatment in a manner appropriate to the indication. Thus, for example, IL-15 compositions administered to suppress a form of anemia can be given by bolus injection, continuous infusion, sustained release from implants, or other suitable technique. Administration may be by intravenous injection, subcutaneous injection, or parenteral or intraperitoneal infusion. Typically, an IL-15 therapeutic agent will be administered in the form of a pharmaceutical composition comprising purified polypeptide in conjunction with physiologically acceptable carriers, excipients or diluents. Such carriers will be nontoxic to patients at the dosages and concentrations employed. Ordinarily, the preparation of such compositions entails combining a mammalian IL-15 polypeptide or derivative thereof with buffers, antioxidants such as ascorbic acid, low molecular weight (less than about 10 residues) polypeptides, proteins, amino acids, carbohydrates including glucose, sucrose or dextrans, chelating agents such as EDTA, glutathione and other stabilizers and excipients. Neutral buffered saline or saline mixed with conspecific serum albumin are exemplary appropriate diluents. The following examples are for purposes of illustration and not by way of limitation.

### EXAMPLE 1

### PURIFICATION AND SEQUENCING FOR NATIVE sIL-15

### Ultrafiltration

Ultrafiltration was not absolutely necessary to purify IL-15. The procedure, however, does remove certain smaller contaminating proteins and reduces the volume, thus speeding up the purification scheme. The ultrafiltration step can be performed using either a YM10 or YM30 spiral cartridge, a hollow fiber cartridge, or a disc membrane in various types of ultrafiltration apparatus. An Amicon ultrafiltration system with a YM30 spiral cartridge, however, was preferred. No buffer exchange was required before or after this step.

A non-homogeneous protein solution, i.e. conditioned medium was obtained by growing CV-1/EBNA cell cultures in serum free, phenol red free DMEM in bioreactors with 5 g/l Cytodex® 3 microcarriers. 8 x 8 liter bioreactors (total of about 64 liters) were harvested, centrifuged to remove cells and microcarriers, filtered through a 0.22 micron cellulose acetate membrane filter, and then concentrated to a final volume of about two liters using a YM30 spiral cartridge. Preferably, the YM30 concentrate is filtered before undergoing hydrophobic chromatography. This step will minimize contamination by removing bacteria and other particulates. Any filter having a pore size from 0.1 to 0.45 microns that does not bind protein may be used; however, a 0.22 micron cellulose acetate membrane filter was preferred.

### Hydrophobic Chromatography

Hydrophobic chromatography provided a quick way of transferring the protein into a low salt buffer that could be applied to anion exchange columns. In addition, it provided three to six fold purification. Still further, no buffer exchange was required before or after this step. Various hydrophobic columns are suitable. A Phenyl Sepharose® CL-4B column (Pharmacia) was preferred. As an alternative to the hydrophobic chromatography step described herein, a diafiltration or dialysis step can be used.

Preferably, ammonium sulfate was added to the ultrafiltration concentrate to a final concentration of about 0.2 M. The ultrafiltration concentrate was buffered with 1M HEPES at about pH 8.5 to a final concentration of about 20mM. The concentrate was then pumped onto a Phenyl Sepharose® CL-4B column and washed with 0.2M ammonium sulfate 10mM HEPES at about pH 8.5 to remove unbound protein. Bound proteins were eluted with 10mM HEPES at about pH 8.5. The eluted protein peak (including IL-15) was applied to an anion exchange column.

### Anion Exchange Chromatography

Anion exchange chromatography purification allowed additional purification without requiring dialysis or buffer exchange. This step preferably involved two passages of the Phenyl Sepharose® protein pool over columns containing an anion exchange media. After each passage, the bound proteins were eluted with NaCl in HEPES. Although various anion exchange media and buffer systems having a pH of about 8 to about 9 were suitable, DEAE Sephacel® (Pharmacia) followed by Mono Q allowed sequential anion exchange steps without buffer exchanges or dialysis. DEAE Sephacel® provided removal of some contaminating proteins prior to application to a higher resolution Mono Q fast performance liquid chromatography ("FPLC," Pharmacia). The NaCl concentration used depended on the anion exchanger selected and the pH of the buffer chosen. Other salts could be substituted for the NaCl to elute the protein from the anion exchange gel.

Most preferably, NaCl was added to the Phenyl Sepharose pool to a final conductivity of about 1.2 milliSiemens/Centimeter ("mS/cm") (less than approximately 0.1 M NaCl) and pumped onto a DEAE Sephacel® column that was equilibrated with about 0.1M NaCl in about 10mM HEPES at about pH 8.5. Bound proteins were eluted with a linear gradient from about 0.1 to about 0.3 M NaCl in about 10mM HEPES at about pH 8.5. The protein active fractions eluted (including IL-15) were pooled for application to a Mono Q anion exchange column.

The active DEAE Sephacel® pool was diluted with about 10 mM HEPES to a final conductivity less than 1.6 mS/cm (less than 0.14 M NaCl). The diluted pool was then pumped onto a Mono Q FPLC fast performance liquid chromatography column that was equilibrated with about 0.14 M NaCl in about 10mM HEPES at about pH 8.5. Bound proteins were eluted with a gradient from about 0.14 M to about 0.5 M NaCl in about 10 mM HEPES at about pH 8.5. The active fractions (including IL-15) were pooled for application to reverse phase high performance liquid chromatography (RP-HPLC).

### RP-HPLC

The native mammalian IL-15 polypeptide is stable from approximately pH 7 to approximately 9 and at approximately pH 2.5 in 0.1% trifluoroacetic acid ("TFA"), in acetonitrile ("AcN"). The IL-15 activity was not recovered when acidic aqueous buffers were used; thus eliminating many HPLC buffer systems and not allowing cation exchange chromatography to be included in the purification scheme. C4 RP-HPLC columns (Vydac™ 0.46 x 25 cm, 5 micron) provided the greatest purification. Other reverse phase columns (C8 or C18) did not work; the protein eluted from C4 at a high AcN concentration and was not recovered from C8 or C18 at all. Other buffer systems that we tried (i.e., ammonium acetate/methanol pH 7 and pyridine acetate/propanol) were not successful.

The RP-HPLC purification preferably involved two passages of the Mono Q active pool over the Vydac™ C4 matrix. In the first passage, the Mono Q active pool was pumped onto a C4 HPLC column at about 1 ml/min and eluted with a gradient of 0.1% TFA/H₂O to 0.1% TFA/100% AcN at the following gradient:
0 to 45% AcN, 1% AcN/minute
45 to 60% AcN, 0.5% AcN/minute
60 to 100% AcN, 2% AcN/minute
Peak active fractions (including IL-15) elute between about 48 and about 51% AcN. Active fractions determined by bioassay were pooled, diluted with 0.1% TFA/H₂O to reduce AcN concentration, and applied to the same C4 column.

The active, diluted pool from the C4 TFA/AcN run was pumped back on to the C4 column, washed with 0.1% TFA/H₂O and eluted with a linear gradient of 0.1% TFA/H₂O (buffer A) to 0.1% TFA/60% n-propanol (buffer B) at about 0.5 ml/min. The gradient was run at about 0.5% buffer B/min. Fractions were bioassayed to identify the IL-15-containing fractions. IL-15-containing fractions were pooled.

### SDS-PAGE

Purified IL-15 can be visualized by silver stained SDS-PAGE. Purified mammalian IL-15 protein bands isolated by SDS-PAGE may be electroblotted and analyzed to determine their N-terminal amino acid sequences. The IL-15 protein band can be identified by bioassay.

Purified IL-15 protein fractions from the C4 TFA/n-propanol HPLC run were speed vacuumed to dryness, resuspended in reducing SDS sample buffer and run on a polyacrylamine SDS gel. Preferably, HPLC purified IL-15 was run on SDS-PAGE (Phastgel® 8-25%, Pharmacia) in two adjacent lanes. Prior to fixing and staining, approximately 1 mm slices of gel were cut from one lane of the Phastgel® and put directly into bioassays. The remaining gel was developed and the silver stained bands matched with slices put into bioassays. The IL-15 activity corresponded to 15-17 kDa. For specific activity determination, purified IL-15 was resuspended in reducing SDS sample buffer, run on 14% polyacrylamide SDS gel (Novex) and silver stained. The purity of the sIL-15 polypeptides corresponding to 15-17 kDa was approximately 222,000 times greater than the sIL-15 polypeptide purity in the CV-1/EBNA conditioned media at the beginning of the purification scheme (Table 2). In addition to purity and protein data, Table 2 shows the activity of the native sIL-15 polypeptide in a CTLL-2 bioassay (described below in Example 2) conducted after each step of the purification process.

### PVDF Blot

The 14% polyacrylamide SDS gel was blotted to a PVDF membrane (ProBlot® from Applied Biosystems) using constant current, at about a 60V setting for about one hour. Protein bands were visualized by staining the PVDF membrane with Coomassie blue (0.1% in 10% acetic acid, 50% methanol). The membrane may be destained using the same solution without the Coomassie blue stain to highlight the protein bands. The protein band corresponding to the

**Table 2**

| **Monkey IL-15 from CV-1/EBNA cell-conditioned serum free media**^{**1**} | | | | | | | |
|---|---|---|---|---|---|---|---|
| SAMPLE | VOLUME | PROTEIN CONC. UG/ML | TOTAL PROTEIN | ACTIVITY UNITS/ML² | TOTAL ACTIVITY UNITS | SPECIFIC ACTIVITY UNITS/UG | FOLD PURIFICATION |
| Crude | 64 L | 40-70 | 3.2 g | <100 | | (1.0) | 1 |
| Phenyl Seph SM | 1.7 L | 2100 | 3.6 g | 3000 | 5,100,000 | 1.4 | 1.4 |
| DEAE SM | 364 ml | 1300 | 473 mg | 5100 | 1,900,000 | 3.9 | 3.9 |
| Mono Q SM | 215 ml | 310 | 66.65 mg | 7664 | 1,650,000 | 24.8 | 25 |
| HPLC TFA-AcN SM | 14 ml | ND³ | ND | 70546 | 987,644 | ND | ND |
| HPLC TFA-prop SM | 4 ml | ND | ND | 220,000 | 880,000 | ND | ND |
| TFA-prop peak | 2 ml | (88) | (176ug) | 440,000 | 888,000 | (5045) | (5000) |
| SDS PAGE band | | | (4 ug) | | | (222,000) | (222,000) |

| | | | | | | | |
|---|---|---|---|---|---|---|---|
| ¹ Values in parenthesis are estimates from silver stained SDS PAGE. Other protein values were determined by Biorad Microprotein Assay (BSA standard) | | | | | | | |
| ² Activity was determined by CTLL-2 bioassay | | | | | | | |
| ³ ND = not determined | | | | | | | |

IL-15 activity was cut out and N-terminal protein sequence determination was performed directly from the PVDF membrane.

### sIL-15 Polypeptide Sequencing

The IL-15 preparation resulting from the foregoing RP-HPLC step was analyzed by SDS-PAGE. Each gel was silver stained to indicated the presence of protein bands. Bioassay of unstained gel slices corresponding to visible bands indicated the IL-15 activity was associated with proteins having molecular weights in the range of 15-17 kDa. The N-terminus of the 15-17 kDa polypeptide, blotted onto a PVDF membrane, was sequenced by Edman degradation in an Applied Biosystems protein sequencer. The results indicated the identity of the first 33 amino acids shown in SEQ ID NO 3. Subsequent sequencing of a cDNA clone obtained from a simian library provided a sequence encoding the polypeptide of SEQ ID NO 2. The polypeptide of SEQ ID NO 2 comprises a relatively short 48 amino acid leader sequence and a mature polypeptide represented by SEQ ID NO 3.

### EXAMPLE 2

### BIOASSAY

CTLL-2 cells provide a fast and sensitive bioassay for detection of IL-15 polypeptides. Other cell lines that also proliferate in response to IL-15 with varying sensitivity are CTLL-2.4 (Valentine et al., *Eur J. Immunol., 21(4:913 (1991*)), 32D (Greenberger*, Federation Proceedings* 42:2762 (1983)), BAF-BO3 (Hatakeyama et al., *Cell, 59*:837 (1989)), MO7e (Avanzi et al., *Br. J. Haematol. 69*:359 (1988)), and TF1 (Kitamura et al., *J. Cell. Physciol*., 140:323 (1989)).

Preferably, CTLL-2 cells were grown in high glucose DMEM supplemented with about 30 ng/ml IL-2, 5% fetal bovine serum (FBS), 5 x 10⁻⁵ M 2-mercaptoethanol, 50 u/ml penicillin, 50 ug/ml streptomycin, and 3-4.0 mM L-glutamine at 37°C, 10% CO₂ and 97% humidity. CTLL-2 is a factor dependent cell line requiring IL-2 for growth. Consequently, for assay, CTLL-2 cells were washed twice with high glucose DMEM supplemented with 5% FBS, 5 x 10⁻⁵ M 2-mercaptoethanol, 50 u/ml penicillin, 50 ug/ml streptomycin, 3-4.0 mM L-glutamine to remove IL-2. IL-15 samples to be assayed were titrated in DMEM 5% FBS in 96 well flat-bottomed microtiter plates. Washed CTLL-2 cells were added (final assay volume 100 µl, 2000 cells/well) and the plates incubated about 24 hours at 37°C and 10% CO₂. The plates were pulsed with ³H-thymidine (25Ci/mMole) at 0.5 µCi/well for about 5 hours, then harvested (Inotech 96 well cell harvester) and CPM counted (Packard Matrix 96 gas proportional counting system). Units were calculated from CPM where 1 unit equals the number of microliters that gives 50% maximal stimulation.

### EXAMPLE 3

### PREPARATION OF sIL-15 cDNA CLONE

The sequence of the N-terminal 31 amino acids of purified sIL-15 polypeptide (amino acids 1-31 in SEQ ID NO 3) was used to design synthetic oligonucleotide primers for PCR amplification of IL-15-specific DNA sequences. The first six amino acids of the N-terminus (Asn-Trp-Val-Asn-Val-Ile) were used to design one primer, a degenerate mixture coding for all possible codon usages of the first six amino acid residues:
5'-AAYTGGGTNAAYGTNATH -3'
as shown in SEQ ID NO 9 where Y is T or C; H is A, T, or C; and N is A, C, G, or T. The amino acid sequences of the simian mature N-terminus 26-31 (Tyr-Thr-Glu-Ser-Asp-Val) were used to design a second primer, a degenerate mixture coding for a complement of all possible codon usages of amino acids 26-31 omitting position 3 of Val:
5'-ACRTCNGAYTCNGTRTA-3' and
5'-ACRTCRCTYTCNGTRTA-3'
as shown in SEQ ID NOS 10 and 11, respectively, where Y and N are as defined above and R is A or G.

Polyadenylated RNAs from CV-1/EBNA cells stimulated for 24 hr, 37 hr, and 72 hr with 10 ng/ml phorbol 12-myristate 13-acetate ("PMA") were used as separate templates for first strand cDNA synthesis. A portion of first strand reactions was added to commercially available PCR reaction mixes containing the oligonucleotide primers. This mixture was subjected to 31 cycles of PCR amplification in 100 µl reactions in standard buffer with the primers at 1µM concentration. The cycles were programmed as follows: denaturation at 94°C for 0.5 min., annealing step for 0.5 min, and elongation step at 72°C for 0.75 min. Two cycles of annealing at each of 55°C, 53°C, and 51°C were followed by 25 cycles with an annealing temperature of 49°C.

Following amplification, samples were purified and subjected to agarose gel electrophoresis. This yielded a 92 base pair DNA fragment that was excised from gel lanes from two separate reactions involving CV-1/EBNA cells. The 92 base pair DNA fragment was purified using an Elutip-D column (Schleicher & Schuell, Keene NH), cloned into pBluescript SK⁻ (Stratagene, La Jolla, CA) and used for dideoxy DNA sequencing.

A hybridization probe was prepared by random prime labeling of the subcloned 92 base pair DNA fragment. The hybridization probe was used to screen a portion of a plasmid library containing cDNA inserts prepared from CV-1/EBNA polyadenylated RNA. This resulted in the isolation of clone C85.sIL-15 that has an open reading frame comprising the nucleotide sequence shown in SEQ ID NO 1.

The nucleotide sequence of the polypeptide coding region of sIL-15 is illustrated in SEQ ID NO 1. This sequence was derived from insert C85.sIL-15 that was *Sal* I linkered into the *Sal* I site of expression vector pDC406 (C. J. McMahan et al., *EMBO J*., 10(10):2821-2832 (1991)). Polyadenylated mRNA was prepared from a CV-1/EBNA cell line and cDNAs were prepared using standard techniques. The CV-1/EBNA line is a producer of sIL-15. cDNA ends were adapted with *Sal* I adapters (Haymerle et al., *Nucleic Acid Res*, 14:8615-24 (1986)): 
5'-TCGACTGGAACGAGACGACCTGCT-3'
3'-GACCTTGCTCTGCTGGACGA-5'
 (SEQ ID NOS 7 and 8, respectively) and cloned into vector pDC406. A pool consisting of approximately 500 individual plasmid-containing isolates was plated and screened by hybridization to a DNA probe fragment. The DNA probe fragment was prepared using PCR amplification of sIL-15 sequences from CV-1/EBNA cell line cDNA.

### EXAMPLE 4

### CLONING HUMAN IL-15

A sIL-15 probe was prepared from an isolated, purified and radiolabeled *Sal* I fragment (about 1.37 kb) containing sIL-15 cDNA by random prime labeling. The specific activity of the probe was approximately 1 x 10⁶ cpm/ng. On Northern blots, the probe was hybridized to human RNAs from various sources, including a IMTLH cell line. The IMTLH cell line was derived from a stable transformation of a human bone marrow stromal cell culture with pSV3Neo. The probe was hybridized to human RNAs at about 42°C in about 40% formamide for about 18 hours. Hybridization was followed by washing in 6 x SSC for about ten minutes at 22°C followed by washing in 2 x SSC at 42°C for about 30 minutes. Autoradiography revealed a positive signal in the IMTLH lane.

We probed Southern blots of *Sal* I-digested library pools of the IMTLH cDNA library to identify a pool containing a human IL-15 cDNA. Using the Haymerle et al., *Nucleic Acid Res*, 14:8615-24 (1986) method used above for the CV-1/EBNA library, the IMTLH library was constructed in expression vector pDC406. Pool "I41", a pool of approximately 1000 different cDNA clones, was identified as positive.. Approximately 4000 colonies of "I41" then were plated and probed by conventional colony hybridization methods to identify a clone containing the human IL-15 cDNA. Only a single clone, I41.hETF, was shown to encode human IL-15. There is approximately 96% nucleotide sequence identity and approximately 96% amino acid sequence identity between human and simian IL-15 open reading frame sequences (Figures 4 and 5).

### EXAMPLE 5

### rIL-15 STIMULATION OF CTLL-2 PROLIFERATION

cDNAs encoding mature forms of IL-15 (nucleotides 145 to 489, inclusive, in SEQ ID NOS 1 and 4) were inserted downstream of a heterologous mammalian secretion signal to create rIL-15 expression plasmid for sIL-15 and hIL-15. The secretion signal is a largely hydrophobic stretch of amino acids (usually at the N-terminus of a polypeptide) that directs secretion and cleavage of the polypeptide between the C-terminus of the signal peptide and the N-terminus of the mature secreted protein (von Heijne, *Eur. J. Biochem*., 116:419 (1981)). The secretion signal sequence used was a murine IL-7 signal sequence. The created plasmids were transfected into COS-7 cells. Supernatants of the transfected cell cultures stimulated CTLL-2 proliferation. In addition, the coding region for the precursor form of hIL-15 (SEQ ID NO 3) was inserted into pDC406 and transfected into CV-1/EBNA cells. Supernatants from cells transfected with pDC406:hIL-15 (i.e.. hETF/pDC406), but not those transfected with empty vector, stimulated CTLL-2 proliferation.

### EXAMPLE 6

### INDUCTION OF CTLL-2 PROLIFERATION BY rIL-15

Recombinant simian IL-15 (simian rIL-15) was purified from supernatant of yeast expressing the sIL-15 cDNA by a modification of the purification method described above in Example 1 in which the untrafiltration and ion exchange steps were omitted. The purified simian rIL-15 was compared to purified recombinant IL-2 and IL-4 for biological activity. The purity of each of the three proteins was confirmed by amino acid analysis. Each of the three purified recombinant proteins was assayed for biological activity *in vitro* using CTLL-2 cells. CTLL-2 cultures contained the indicated cytokine with 2000 cells/culture in 100 µl of supplemented culture medium. The CTLL-2 cultures were pulsed with 0.5 µCi [3H]thymidine/culture for the last 4 hours of a 24 hour culture period, harvested onto glass fiber filter and radioactivity was determined by avalanche gas ionization. The *in vitro* proliferative response of CTLL-2 cells was measured at increasing concentrations of recombinant cytokine (expressed as ng/ml). The data are expressed as cpm of ³H-thymidine incorporated (X 10⁻³) in Figure 6.

### EXAMPLE 7

### INDUCTION OF CTL, LAK, AND NK LYTIC ACTIVITY BY rIL-15

Antigen specific cytolytic T lymphocytes (CTL) were generated *in vitro*. Human peripheral blood mononuclear cells (PBL) from one donor (5 x 10⁵/culture) were stimulated with irradiated PBL (5 x 10⁵/culture) from an allogeneic donor in cultures containing various concentrations of either IL-2 or human rIL-15, or no cytokine. Cultures were performed as described by M.B. Widmer *et al*. in *J. Exp. Med*., 166:1447 (1987), harvested after 7 days, and assayed for cytolytic activity against ⁵¹Cr labeled targets derived from the original stimulating donor. The lysis assay contained various numbers of the responding PBL cultured with 1000 labeled targets in 200 µl of medium in V-bottomed wells, and supernatants were collected after 4 hours of incubation. Lytic units were calculated as the inverse of the fraction of the responding culture required to generate 50% of the maximum specific ⁵¹Cr release. The data for the cytokine-treated CTL are summarized in Figure 7.

Lymphokine activated killer (LAK) cells were generated under identical culture conditions as the CTL described above except that the human PBL were not stimulated with irradiated PBL from an allogeneic donor. Instead, irradiated autologous PBL were substituted and cytolytic activity was measured against the Daudi lymphoblastoid cell line. For the LAK assay, lytic units were calculated as the inverse of the fraction of the responding culture required to generate 30% of the maximum specific ⁵¹Cr release. The data for the cytokine-treated LAK cells are summarized in Figure 8.

Natural killer (NK) cells were isolated from whole human PBL isolated by antibody affinity to paramagnetic microspheres with MACS (Miltenyi Biotec, Sunnyvale CA) using monoclonal antibodies against CD16. The purified NK cells were cultured for 3 days and cytolytic activity was measured against the K562 erythroleukemia cell line. For the NK assay, lytic units were calculated as the inverse of the fraction of the responding culture required to generate 30% of the maximum specific ⁵¹Cr release. The data for the cytokine-treated NK cells are summarized in Figure 9.

Due to the similarity in activity between IL-2 and IL-15 in Examples 6 and 7 hereof, one of ordinary skill in the art would expect IL-15 to stimulate the activity of CTL, LAK and NK cells and expand the population of T cells that can destroy tumor cells and viral-infected cells. As described above in the section on Administration of the IL-15 polypeptide, an effective amount of IL-15 in a suitable diluent or carrier can be administered to patients with carcinomas, melanomas, sarcomas leukemia or lymphomas, or patients infected with Herpesviridae, including cytomegalovirus, Polyomaviridae, Retroviridae, including HIV, influenza virus, Hepadnaviridae, hepatitis A, hepatitis B, hepatitis C, hepatitis delta, or hepatitis D.

## Claims

1. An isolated DNA sequence encoding a polypeptide exhibiting mammalian interleukin-15 (IL-15) biological activity which is capable of stimulating T-lymphocyte proliferation and/or differentiation, wherein the DNA sequence is selected from the group consisting of:
(a) a DNA sequence encoding a mammalian IL-15 polypeptide comprising the sequence: 
Asn Trp Val Asn Val Ile Ser Asp Leu Lys Lys Ile Glu Asp Leu Ile
1               5                   10                  15

Gln Ser Met His Ile Asp Ala Thr Leu Tyr Thr Glu Ser Asp Val His
            20                  25                  30

Pro Ser Cys Lys Val Thr Ala Met Lys Cys Phe Leu Leu Glu Leu Gln
        35                  40                  45

Val Ile Ser Xaa Glu Ser Gly Asp Xaa Xaa Ile His Asp Thr Val Glu
    50                  55                  60

Asn Leu Ile Ile Leu Ala Asn Asn Xaa Leu Ser Ser Asn Gly Asn Xaa
65                  70                  75                  80

Thr Glu Ser Gly Cys Lys Glu Cys Glu Glu Leu Glu Glu Lys Asn Ile
                85                  90                  95

Lys Glu Phe Leu Gln Ser Phe Val His Ile Val Gln Met Phe Ile Asn
            100                 105                 110

Thr Ser
 wherein amino acid 52 is Leu or His, amino acid 57 is Ala or Thr, amino acid 58 is Ser or Asp, amino acid 73 is Ser or Ile, and amino acid 80 is Val or Ile; and
(b) DNA sequences that detectably hybridize to the DNA sequences of (a) or their complementary strands under conditions of high stringency and code on expression for a polypeptide with mammalian IL-15 biological activity.

2. An isolated DNA sequence according to claim 1, wherein said DNA sequence encoding a polypeptide exhibiting mammalian IL-15 activity comprises a DNA sequence selected from the group consisting of:
(a) nucleotides 145 through 489 of the DNA sequence of SEQ ID NO 1;
(b) DNA sequences tat detectably hybridize to the DNA sequences of (a) or their complementary strands under conditions of high stringency and code on expression for a polypeptide with mammalian IL-15 biological activity; and
(c) DNA sequences that, due to degeneracy of the genetic code, encode a polypeptide encoded by any of the foregoing DNA sequences.

3. An isolated DNA sequence according to claim 1, wherein said DNA sequence encoding a polypeptide exhibiting mammalian IL-15 activity comprises a DNA sequence selected from the group consisting of:
(a) nucleotides 145 through 489 of the DNA sequence of SEQ ID NO 4;
(b) DNA sequences that detectably hybridize to the DNA sequences of (a) or their complementary strands under conditions of high stringency and code on expression for a polypeptide with mammalian IL-15 biological activity; and
(c) DNA sequences that, due to degeneracy of the genetic code, encode a polypeptide encoded by any of the foregoing DNA sequences.

4. A recombinant expression vector comprising a DNA sequence encoding a polypeptide with mammalian IL-15 biological activity according to claim 1.

5. A recombinant expression vector according to claim 4, wherein said DNA sequence encoding a polypeptide exhibiting mammalian IL-15 biological activity is selected from the groups consisting of:
(a) nucleotides 145 through 489 the DNA sequence of SEQ ID NO 1;
(b) nucleotides 145 through 489 the DNA sequence of SEQ ID NO 4;
(c) DNA sequences that detectably hybridize to the DNA sequences of (a) or (b) or their complementary strands under conditions of high stringency and code on expression for a polypeptide with mammalian IL-15 biological activity; and
(d) DNA sequences that, due to degeneracy of the genetic code, encode a polypeptide a polypeptide encoded by any of the foregoing DNA sequences.

6. A host cell transformed or transfected with an expression vector according to claim 4 or claim 5.

7. The host cell of claim 6 wherein the host cell is selected from the group consisting of *E. coli, Pseudomonas, Bacillus, Streptomyces,* yeast, fungi, insect cells and mammalian cells.

8. The host cell of claim 7 wherein the yeast host cell is *Saccharomyces cerevisiae*.

9. The host cell of claim 7 wherein the mammalian host cell is Chinese hamster ovary.

10. A process for preparing an IL-15 polypeptide, comprising culturing a host cell according to claim 6 or claim 7 under conditions promoting expression and recovering a polypeptide exhibiting IL-15 biological activity from the culture.

11. An isolated biologically active IL-15 polypeptide composition which is capable of stimulating T-lymphocyte proliferation and/or differentiation, comprising an amino acid sequence encoded by an isolated DNA sequence according to claim 1, the polypeptide defined by SEQ ID NO 3, the polypeptide defined by SEQ ID NO 2, the polypeptide defined by SEQ ID NO 6 or the polypeptide defined by SEQ ID NO 5.

12. A pharmaceutical composition for stimulating proliferation of T-lymphocytes comprising the isolated IL-15 polypeptide of claim 11.

13. An isolated DNA sequence encoding a precursor polypeptide of the biologically active IL-15 polypeptide which is capable of stimulating T-lymphocyte proliferation and/or differentiation, wherein said DNA sequence is selected from the group consisting of:
a DNA sequence encoding on expression the polypeptide defined by SEQ ID NO 2; and
a DNA sequence encoding on expression the polypeptide defined by SEQ ID NO 5.

14. The isolated DNA sequence according to claim 13, wherein said DNA sequence encoding a precursor polypeptide of the biologically active IL-15 polypeptide is selected from the group consisting of:
the DNA sequence of SEQ ID NO 1; and
the DNA sequence of SEQ ID NO 4.

15. A polypeptide as defined in claim 11 for use in medicine.

16. The use of a polypeptide as defined in claim 11 in the preparation of an agent for the treatment or prevention of anaemia, carcinoma, melanoma, sarcoma, leukaemia, lymphoma, or for the treatment or prevention of infections caused by Herpesviridae, including Cytomegalovirus, Polyomaviridae, Retroviridae, including HIV, influenza virus, Hepadnaviridae, including hepatitis A, hepatitis B, hepatitis C, hepatitis delta or hepatitis D.

## Patentansprüche

1. Isolierte DNA-Sequenz, die für ein Polypeptid kodiert, das biologische Säugetier-Interleukin-15- (IL-15) Aktivität aufweist das die T-Lymphozyten-Proliferation und/oder Differenzierung stimulieren kann, wobei die DNA Sequenz ausgewählt ist aus der Gruppe bestehend aus:
(a) einer DNA-Sequenz, die für ein Säugetier-IL-15-Polypepid kodiert, mit der Sequenz: 
Asn Trp Val Asn Val Ile Ser Asp Leu Lys Lys Ile Glu Asp Leu Ile
1               5                   10                  15

Gln Ser Met His Ile Asp Ala Thr Leu Tyr Thr Glu Ser Asp Val His
            20                  25                  30

Pro Ser Cys Lys Val Thr Ala Met Lys Cys Phe Leu Leu Glu Leu Gln
        35                  40                  45

Val Ile Ser Xaa Glu Ser Gly Asp Xaa Xaa Ile His Asp Thr Val Glu
    50                  55                  60

Asn Leu Ile Ile Leu Ala Asn Asn Xaa Leu Ser Ser Asn Gly Asn Xaa
65                  70                  75                  80

Thr Glu Ser Gly Cys Lys Glu Cys Glu Glu Leu Glu Glu Lys Asn Ile
                85                  90                  95

Lys Glu Phe Leu Gln Ser Phe Val His Ile Val Gln Met Phe Ile Asn
            100                 105                 110

Thr Ser
 wobei die Aminosäure 52 Leu oder His ist die Aminosäure 57 Ala oder Thr ist die Aminosäure 58 Ser oder Asp ist, die Aminosäure 73 Ser oder Ile ist und die Aminosäure 80 Val oder Ile ist; und
(b) DNA-Sequenzen, die nachweisbar an die DNA-Sequenzen von (a) oder deren komplementäre Stränge unter stringenten Bedingungen hybridisieren und bei Expression für ein Polypeptid mit biologischer Säugetier-IL-15-Aktivität kodieren.

2. Isolierte DNA-Sequenz nach Anspruch 1, wobei die DNA-Sequenz, die für ein Polypeptid kodiert, das Säugetier-IL-15-Aktivität aufweist eine DNA-Sequenz aufweist, die ausgewählt ist aus der Gruppe bestehend aus:
(a) Nukleotiden 145 bis 489 der DNA-Sequenz von SEQ ID NR 1;
(b) DNA-Sequenzen, die nachweisbar an die DNA-Sequenzen von (a) oder deren komplementäre Stränge unter stringenten Bedingungen hybridisieren und bei Expression für ein Polypeptid mit biologischer Säugetier-IL-15-Aktivität kodieren; und
(c) DNA-Sequenzen, die aufgrund einer Degeneration des genetischen Kodes für ein Polypeptid kodieren, das von einer der vorangehenden DNA-Sequenzen kodiert wird.

3. Isolierte DNA-Sequenz nach Anspruch 1, wobei die DNA-Sequenz, die für ein Polypeptid kodiert, das Säugetier-IL-15-Aktivität aufweist, eine DNA-Sequenz aufweist, die ausgewählt ist aus der Gruppe bestehend aus:
(a) Nukleotiden 145 bis 489 der DNA-Sequenz von SEQ ID NR 4;
(b) DNA-Sequenzen, die nachweisbar an die DNA-Sequenzen von (a) oder deren komplementäre Stränge unter stringenten Bedingungen hybridisieren und bei Expression für ein Polypeptid mit biologischer Säugetier-IL-15-Aktivität kodieren; und
(c) DNA-Sequenzen, die aufgrund einer Degeneration des genetischen Kodes für ein Polypeptid kodieren, das von einer der vorangehenden DNA-Sequenzen kodiert wird.

4. Rekombinanter Expressionsvektor, der eine DNA-Sequenz aufweist, die für ein Polypeptid mit biologischer Säugetier-IL-15-Aktivität kodiert, nach Anspruch 1.

5. Rekombinanter Expressionsvektor nach Anspruch 4, wobei die DNA-Sequenz, die für ein Polypeptid kodiert, das Säugetier-IL-15-Aktivität aufweist, ausgewählt ist aus der Gruppe bestehend aus:
(a) Nukleotiden 145 bis 489 der DNA-Sequenz von SEQ ID NR 1;
(b) Nukleotiden 145 bis 489 der DNA-Sequenz von SEQ ID NR 4;
(c) DNA-Sequenzen, die nachweisbar an die DNA-Sequenzen von (a) oder (b) oder deren komplementäre Stränge unter stringenten Bedingungen hybridisieren und bei Expression für ein Polypeptid mit biologischer Säugetier-IL-15-Aktivität kodieren und
(d) DNA-Sequenzen, die aufgrund einer Degeneration des genetischen Kodes für ein Polypeptid kodieren, das von einer der vorangehenden DNA-Sequenzen kodiert wird.

6. Wirtszelle, die mit einem Expressionsvektor nach Anspruch 4 oder Anspruch 5 transformiert oder transfektiert ist.

7. Wirtszelle nach Anspruch 6, wobei die Wirtszelle ausgewählt ist aus der Gruppe bestehend aus *E. coli, Pseudomonas, Bacillus, Streptomyces,* Hefe, Pilzen, Insektenzellen und Säugetierzellen.

8. Wirtszelle nach Anspruch 7, wobei die Hefewirtszelle *Saccharomyces cerevisiae* ist.

9. Wirtszelle nach Anspruch 7, wobei die Säugetierwirtszelle die Ovarialzelle des chinesischen Hamsters ist.

10. Verfahren zur Herstellung eines IL-15 Peptids, der das Züchten einer Wirtszelle nach Anspruch 6 oder Anspruch 7, unter Bedingungen, welche die Expression fördern, und das Gewinnen eines Polypeptids, das biologische IL-15-Aktivität aufweist, aus der Kultur umfaßt.

11. Isolierte, biologisch aktive IL-15 Polypeptidzusammensetzung, welche die T-Lymphozyten-Proliferation und/oder Differenzierung stimulieren kann, mit einer Aminosäuresequenz, die von einer isolierten DNA-Sequenz nach Anspruch 1 kodiert wird, dem Polypeptid, das durch die SEQ ID NR 3 definiert ist, dem Polypeptid, das durch die SEQ ID NR 2 definiert ist, dem Polypeptid, das durch die SEQ ID NR 6 definiert ist, oder dem Polypeptid, das durch die SEQ ID Nr.5 definiert ist.

12. Pharmazeutische Zusammensetzung zur Stimulierung der Proliferation von T-Lymphozyten, welche das isolierte IL-15-Polypeptid nach Anspruch 11 enthält.

13. Isolierte DNA-Sequenz, die für ein Vorläuferpolypeptid des biologisch aktiven IL-15-Polypeptids kodiert, das die T-Lymphozyten-Proliferation und/oder Differenzierung stimulieren kann, wobei die DNA-Sequenz ausgewählt ist aus der Gruppe bestehend aus:
einer DNA-Sequenz, die bei Expression das Polypeptid kodiert, das durch die SEQ ID NR 2 definiert ist; und
einer DNA-Sequenz, die bei Expression das Polypeptid kodiert, das durch die SEQ ID NR 5 definiert ist.

14. Isolierte DNA-Sequenz nach Anspruch 13, wobei die DNA-Sequenz, die für ein Vorläuferpolypeptid des biologisch aktiven IL-15-Polypeptids kodiert, ausgewählt ist aus der Gruppe bestehend aus:
der DNA-Sequenz von SEQ ID NR 1; und
der DNA-Sequenz von SEQ ID NR 4.

15. Polypeptid nach Anspruch 11 zur Verwendung in der Medizin.

16. Verwendung eines Polypeptids nach Anspruch 11 in der Herstellung eines Mittels zur Behandlung oder Vermeidung von Anämie, Karzinom, Melanom, Sarkom, Leukämie, Lymphom, oder zur Behandlung oder Vermeidung von Infektionen, die durch Herpesviridae, einschließlich Cytomegalovirus, Polyomaviridae, Retroviridae, einschließlich HIV, Influenzavirus, Hepadnaviridae, einschließlich Hepatitis A, Hepatitis B, Hepatitis C, Hepatitis Delta oder Hepatitis D, verursacht werden.

## Revendications

1. Séquence isolée d'ADN codant pour un polypeptide montrant une activité biologique d'interleukine-15 (IL-15) mammalienne qui peut stimuler une prolifération et/ou une différenciation de lymphocytes T, la séquence d'ADN étant sélectionnée parmi le groupe contenant :
(a) une séquence d'ADN codant pour un polypeptide IL-15 mammalien comprenant la séquence : 
Asn Trp Val Asn Val Ile Ser Asp Leu Lys Lys Ile Glu Asp Leu Ile
1               5                   10                  15
Gln Ser Met His Ile Asp Ala Thr Leu Tyr Thr Glu Ser Asp Val His
            20                  25                  30
Pro Ser Cys Lys Val Thr Ala Met Lys Cys Phe Leu Leu Glu Leu Gln
        35                  40                  45
Val Ile Ser Xaa Glu Ser Gly Asp Xaa Xaa Ile His Asp Thr Val Glu
    50                  55                  60
Asn Leu Ile Ile Leu Ala Asn Asn Xaa Leu Ser Ser Asn Gly Asn Xaa
65                  70                  75                  80
Thr Glu Ser Gly Cys Lys Glu Cys Glu Glu Leu Glu Glu Lys Asn Ile
                85                  90                  95
Lys Glu Phe Leu Gln Ser Phe Val His Ile Val Gln Met Phe Ile Asn
            100                 105                 110
Thr Ser
 dans laquelle l'acide aminé 52 est Leu ou His, l'acide aminé 57 est Ala ou Thr, l'acide aminé 58 est Ser ou Asp, l'acide aminé 73 est Ser ou Ile, et l'acide aminé 80 est Val ou Ile; et
(b) des séquences d'ADN qui s'hybrident de manière détectable aux séquences d'ADN de (a) ou à leurs brins complémentaires dans des conditions très sévères et codent pour l'expression d'un polypeptide avec une activité biologique d'IL-15 mammalienne.

2. Séquence isolée d'ADN suivant la revendication 1, dans laquelle ladite séquence d'ADN codant pour un polypeptide montrant une activité d'IL-15 mammalienne comprend une séquence d'ADN sélectionnée parmi le groupe contenant :
(a) les nucléotides 145 à 489 de la séquence d'ADN de SEQ ID n°1;
(b) des séquences d'ADN qui s'hybrident de manière détectable aux séquences d'ADN de (a) ou à leurs brins complémentaires dans des conditions très sévères et codent pour l'expression d'un polypeptide avec une activité biologique d'IL-15 mammalienne; et
(c) des séquences d'ADN qui, en raison d'une dégénérescence du code génétique, codent pour un polypeptide codé par une quelconque des séquences d'ADN précédentes.

3. Séquence isolée d'ADN suivant la revendication 1, dans laquelle ladite séquence d'ADN codant pour un polypeptide montrant une activité d'IL-15 mammalienne comprend une séquence d'ADN sélectionnée parmi le groupe contenant :
(a) les nucléotides 145 à 489 de la séquence d'ADN de SEQ ID n°4;
(b) des séquences d'ADN qui s'hybrident de manière détectable aux séquences d'ADN de (a) ou à leurs brins complémentaires dans des conditions très sévères et codent pour l'expression d'un polypeptide avec une activité biologique d'IL-15 mammalienne; et
(c) des séquences d'ADN qui, en raison d'une dégénérescence du code génétique, codent pour un polypeptide codé par une quelconque des séquences d'ADN précédentes.

4. Vecteur d'expression recombiné comprenant une séquence d'ADN codant pour un polypeptide avec une activité biologique d'IL-15 mammalienne suivant la revendication 1.

5. Vecteur d'expression recombiné suivant la revendication 4, dans lequel ladite séquence d'ADN codant pour un polypeptide montrant une activité biologique d'IL-15 mammalienne est sélectionnée parmi le groupe contenant :
(a) les nucléotides 145 à 489 de la séquence d'ADN de SEQ ID n°1;
(b) les nucléotides 145 à 489 de la séquence d'ADN de SEQ ID n°4;
(c) des séquences d'ADN qui s'hybrident de manière détectable aux séquences d'ADN de (a) ou de (b) ou à leurs brins complémentaires dans des conditions très sévères et codent pour l'expression d'un polypeptide avec une activité biologique d'IL-15 mammalienne; et
(d) des séquences d'ADN qui, en raison d'une dégénérescence du code génétique, codent pour un polypeptide codé par une quelconque des séquences d'ADN précédentes.

6. Cellule hôte transformée ou transfectée avec un vecteur d'expression suivant la revendication 4 ou la revendication 5.

7. Cellule hôte suivant la revendication 6, dans laquelle la cellule hôte est sélectionné parmi le groupe contenant *E. coli, Pseudomonas, Bacillus*, *Streptomyces*, levure, champignons, cellules d'insectes et cellules mammaliennes.

8. Cellule hôte suivant la revendication 7, dans laquelle la cellule hôte de levure est *Saccharomyces cerevisiae*.

9. Cellule hôte suivant la revendication 7, dans laquelle la cellule hôte mammalienne provient d'un ovaire d'hamster chinois.

10. Procédé pour préparer un polypeptide IL-15, comprenant la culture d'une cellule hôte suivant la revendication 6 ou la revendication 7 dans des conditions favorisant l'expression et à la récupération d'un polypeptide montrant une activité biologique d'IL-15 à partir de la culture.

11. Composition de polypeptide IL-15 isolé biologiquement actif qui peut stimuler une prolifération et/ou une différenciation de lymphocytes T, comprenant une séquence d'acides aminés codée par une séquence isolée d'ADN suivant la revendication 1, le polypeptide défini par SEQ ID n° 3, le polypeptide défini par SEQ ID n° 2, le polypeptide défini par SEQ ID n° 6 ou le polypeptide défini par SEQ ID n° 5.

12. Composition pharmaceutique pour stimuler la prolifération de lymphocytes T, comprenant le polypeptide IL-15 isolé de la revendication 11.

13. Séquence isolée d'ADN codant pour un polypeptide précurseur du polypeptide IL-15 biologiquement actif qui peut stimuler une prolifération et/ou une différenciation de lymphocytes T, la séquence d'ADN étant sélectionnée parmi le groupe contenant :
une séquence d'ADN codant pour l'expression du polypeptide défini par SEQ ID n° 2; et
une séquence d'ADN codant pour l'expression du polypeptide défini par SEQ ID n° 5.

14. Séquence isolée d'ADN suivant la revendication 13, dans laquelle ladite séquence d'ADN codant pour un polypeptide précurseur du polypeptide IL-15 biologiquement actif est sélectionnée parmi le groupe contenant :
la séquence d'ADN de SEQ ID n° 1; et
la séquence d'ADN de SEQ ID n° 4.

15. Polypeptide comme défini à la revendication 11, pour une utilisation en médecine.

16. Utilisation d'un polypeptide comme défini à la revendication 11, dans la préparation d'un agent pour le traitement ou la prévention de l'anémie, du carcinome, du mélanome, du sarcome, de la leucémie, du lymphome, ou pour le traitement ou la prévention d'infections causées par des Herpèsviridae, y compris le cytomégalovirus, des polyomaviridae, des Rétroviridae, y compris le HIV, le virus de l'influenza, des Hepadnaviridae y compris l'hépatite A, l'hépatite B, l'hépatite C, l'hépatite delta ou l'hépatite D.


SEQUENCE LISTING

(1) GENERAL INFORMATION:

(i) APPLICANT: Grabstein, Kenneth
Anderson, Dirk
Eisenman, June
Fung, Victor
Rauch, Charles

(ii) TITLE OF INVENTION: Interleukin-15

(iii) NUMBER OF SEQUENCES: 12

(iv) CORRESPONDENCE ADDRESS:
(A) ADDRESSEE: Immunex Corporation
(B) STREET: 51 University Street
(C) CITY: Seattle
(D) STATE: Washington
(E) COUNTRY: USA
(F) ZIP: 98101

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25

(vi) CURRENT APPLICATION DATA:
(A) APPLICATION NUMBER:
(B) FILING DATE:
(C) CLASSIFICATION:

(viii) ATTORNEY/AGENT INFORMATION:
(A) NAME: Launer, Charlene
(B) REGISTRATION NUMBER: 33,035
(C) REFERENCE/DOCKET NUMBER: 2811

(ix) TELECOMMUNICATION INFORMATION:
(A) TELEPHONE: 206-587-0430

(2) INFORMATION FOR SEQ ID NO:1:

(i) SEQUENCE CHARACTERISTICS:
(A) LENGTH: 489 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iv) ANTI-SENSE: NO

(ix) FEATURE:
(A) NAME/KEY: CDS
(B) LOCATION: 1..489

(xi) SEQUENCE DESCRIPTION: SEQ ID NO:1:

ATG AGA ATT TCG AAA CCA CAT TTG AGA AGT ATT TCC ATC CAG TGC TAC 48
Met Arg Ile Ser Lys Pro His Leu Arg Ser Ile Ser Ile Gln Cys Tyr
1               5                   10                  15

CTG TGT TTA CTT CTA AAG AGT CAT TTT CTA ACT GAA GCT GGC ATT CAT 96
Leu Cys Leu Leu Leu Lys Ser His Phe Leu Thr Glu Ala Gly Ile His
            20                  25                  30

GTC TTC ATT TTG GGC TGT TTC AGT GCA GGG CTC CCT AAA ACA GAA GCC 144
Val Phe Ile Leu Gly Cys Phe Ser Ala Gly Leu Pro Lys Thr Glu Ala
        35                  40                  45

AAC TGG GTG AAT GTA ATA AGT GAT TTG AAA AAA ATT GAA GAT CTT ATT 192
Asn Trp Val Asn Val Ile Ser Asp Leu Lys Lys Ile Glu Asp Leu Ile
    50                  55                  60

CAA TCT ATG CAT ATT GAT GCT ACT TTA TAT ACA GAA AGT GAT GTT CAC 240
Gln Ser Met His Ile Asp Ala Thr Leu Tyr Thr Glu Ser Asp Val His
65                  70                  75                  80

CCC AGT TGC AAG GTA ACA GCA ATG AAG TGC TTT CTC TTG GAG TTG CAA 288
Pro Ser Cys Lys Val Thr Ala Met Lys Cys Phe Leu Leu Glu Leu Gln
                85                  90                  95

GTT ATT TCA CAT GAG TCC GGA GAT ACA GAT ATT CAT GAT ACA GTA GAA 336
Val Ile Ser His Glu Ser Gly Asp Thr Asp Ile His Asp Thr Val Glu
            100                 105                 110

AAT CTT ATC ATC CTA GCA AAC AAC ATC TTG TCT TCT AAT GGG AAT ATA 384
Asn Leu Ile Ile Leu Ala Asn Asn Ile Leu Ser Ser Asn Gly Asn Ile
        115                 120                 125

ACA GAA TCT GGA TGC AAA GAA TGT GAG GAA CTA GAG GAA AAA AAT ATT 432
Thr Glu Ser Gly Cys Lys Glu Cys Glu Glu Leu Glu Glu Lys Asn Ile
    130                 135                 140

AAA GAA TTT TTG CAG AGT TTT GTA CAT ATT GTC CAA ATG TTC ATC AAC 480
Lys Glu Phe Leu Gln Ser Phe Val His Ile Val Gln Met Phe Ile Asn
145                 150                 155                 160

ACT TCT TGA 489
Thr Ser

(2) INFORMATION FOR SEQ ID NO:2:

(i) SEQUENCE CHARACTERISTICS:
(A) LENGTH: 162 amino acids
(B) TYPE: amino acid
(D) TOPOLOGY: linear

(ii) MOLECULE TYPE: protein

(xi) SEQUENCE DESCRIPTION: SEQ ID NO:2:

Met Arg Ile Ser Lys Pro His Leu Arg Ser Ile Ser Ile Gln Cys Tyr
1               5                   10                  15

Leu Cys Leu Leu Leu Lys Ser His Phe Leu Thr Glu Ala Gly Ile His
            20                  25                  30

Val Phe Ile Leu Gly Cys Phe Ser Ala Gly Leu Pro Lys Thr Glu Ala
        35                  40                  45

Asn Trp Val Asn Val Ile Ser Asp Leu Lys Lys Ile Glu Asp Leu Ile
    50                  55                  60

Gln Ser Met His Ile Asp Ala Thr Leu Tyr Thr Glu Ser Asp Val His
65                  70                  75                  80

Pro Ser Cys Lys Val Thr Ala Met Lys Cys Phe Leu Leu Glu Leu Gln
                85                  90                  95

Val Ile Ser His Glu Ser Gly Asp Thr Asp Ile His Asp Thr Val Glu
            100                 105                 110

Asn Leu Ile Ile Leu Ala Asn Asn Ile Leu Ser Ser Asn Gly Asn Ile
        115                 120                 125

Thr Glu Ser Gly Cys Lys Glu Cys Glu Glu Leu Glu Glu Lys Asn Ile
    130                 135                 140

Lys Glu Phe Leu Gln Ser Phe Val His Ile Val Gln Met Phe Ile Asn
145                 150                 155                 160

Thr Ser

(2) INFORMATION FOR SEQ ID NO:3:

(i) SEQUENCE CHARACTERISTICS:
(A) LENGTH: 114 amino acids
(B) TYPE: amino acid
(D) TOPOLOGY: linear

(ii) MOLECULE TYPE: protein

(iii) HYPOTHETICAL: NO

(xi) SEQUENCE DESCRIPTION: SEQ ID NO:3:

Asn Trp Val Asn Val Ile Ser Asp Leu Lys Lys Ile Glu Asp Leu Ile
1               5                   10                  15

Gln Ser Met His Ile Asp Ala Thr Leu Tyr Thr Glu Ser Asp Val His
            20                  25                  30

Pro Ser Cys Lys Val Thr Ala Met Lys Cys Phe Leu Leu Glu Leu Gln
        35                  40                  45

Val Ile Ser His Glu Ser Gly Asp Thr Asp Ile His Asp Thr Val Glu
    50                  55                  60

Asn Leu Ile Ile Leu Ala Asn Asn Ile Leu Ser Ser Asn Gly Asn Ile
65                  70                  75                  80

Thr Glu Ser Gly Cys Lys Glu Cys Glu Glu Leu Glu Glu Lys Asn Ile
                85                  90                  95

Lys Glu Phe Leu Gln Ser Phe Val His Ile Val Gln Met Phe Ile Asn
            100                 105                 110

Thr Ser

(2) INFORMATION FOR SEQ ID NO:4:

(i) SEQUENCE CHARACTERISTICS:
(A) LENGTH: 489 base pairs
(B) TYPE: nucleic acid
(C) STRANDEDNESS: single
(D) TOPOLOGY: linear

(ii) MOLECULE TYPE: cDNA

(ix) FEATURE:
(A) NAME/KEY: CDS
(B) LOCATION: 1..489

(xi) SEQUENCE DESCRIPTION: SEQ ID NO:4:

ATG AGA ATT TCG AAA CCA CAT TTG AGA AGT ATT TCC ATC CAG TGC TAC 48
Met Arg Ile Ser Lys Pro His Leu Arg Ser Ile Ser Ile Gln Cys Tyr
1               5                   10                  15

TTG TGT TTA CTT CTA AAC AGT CAT TTT CTA ACT GAA GCT GGC ATT CAT 96
Leu Cys Leu Leu Leu Asn Ser His Phe Leu Thr Glu Ala Gly Ile His
            20                  25                  30

GTC TTC ATT TTG GGC TGT TTC AGT GCA GGG CTT CCT AAA ACA GAA GCC 144
Val Phe Ile Leu Gly Cys Phe Ser Ala Gly Leu Pro Lys Thr Glu Ala
        35                  40                  45

AAC TGG GTG AAT GTA ATA AGT GAT TTG AAA AAA ATT GAA GAT CTT ATT 192
Asn Trp Val Asn Val Ile Ser Asp Leu Lys Lys Ile Glu Asp Leu Ile
    50                  55                  60

CAA TCT ATG CAT ATT GAT GCT ACT TTA TAT ACG GAA AGT GAT GTT CAC 240
Gln Ser Met His Ile Asp Ala Thr Leu Tyr Thr Glu Ser Asp Val His
65                  70                  75                  80

CCC AGT TGC AAA GTA ACA GCA ATG AAG TGC TTT CTC TTG GAG TTA CAA 288
Pro Ser Cys Lys Val Thr Ala Met Lys Cys Phe Leu Leu Glu Leu Gln
                85                  90                  95

GTT ATT TCA CTT GAG TCC GGA GAT GCA AGT ATT CAT GAT ACA GTA GAA 336
Val Ile Ser Leu Glu Ser Gly Asp Ala Ser Ile His Asp Thr Val Glu
            100                 105                 110

AAT CTG ATC ATC CTA GCA AAC AAC AGT TTG TCT TCT AAT GGG AAT GTA 384
Asn Leu Ile Ile Leu Ala Asn Asn Ser Leu Ser Ser Asn Gly Asn Val
        115                 120                 125

ACA GAA TCT GGA TGC AAA GAA TGT GAG GAA CTG GAG GAA AAA AAT ATT 432
Thr Glu Ser Gly Cys Lys Glu Cys Glu Glu Leu Glu Glu Lys Asn Ile
    130                 135                 140

AAA GAA TTT TTG CAG AGT TTT GTA CAT ATT GTC CAA ATG TTC ATC AAC 480
Lys Glu Phe Leu Gln Ser Phe Val His Ile Val Gln Met Phe Ile Asn
145                 150                 155                 160

ACT TCT TGA                                                                 489
Thr Ser

(2) INFORMATION FOR SEQ ID NO:5:

(i) SEQUENCE CHARACTERISTICS:
(A) LENGTH: 162 amino acids
(B) TYPE: amino acid
(D) TOPOLOGY: linear

(ii) MOLECULE TYPE: protein

(xi) SEQUENCE DESCRIPTION: SEQ ID NO:5:

Met Arg Ile Ser Lys Pro His Leu Arg Ser Ile Ser Ile Gln Cys Tyr
1               5                   10                  15

Leu Cys Leu Leu Leu Asn Ser His Phe Leu Thr Glu Ala Gly Ile His
            20                  25                  30

Val Phe Ile Leu Gly Cys Phe Ser Ala Gly Leu Pro Lys Thr Glu Ala
        35                  40                  45

Asn Trp Val Asn Val Ile Ser Asp Leu Lys Lys Ile Glu Asp Leu Ile
    50                  55                  60

Gln Ser Met His Ile Asp Ala Thr Leu Tyr Thr Glu Ser Asp Val His
65                  70                  75                  80

Pro Ser Cys Lys Val Thr Ala Met Lys Cys Phe Leu Leu Glu Leu Gln
                85                  90                  95

Val Ile Ser Leu Glu Ser Gly Asp Ala Ser Ile His Asp Thr Val Glu
            100                 105                 110

Asn Leu Ile Ile Leu Ala Asn Asn Ser Leu Ser Ser Asn Gly Asn Val
        115                 120                 125

Thr Glu Ser Gly Cys Lys Glu Cys Glu Glu Leu Glu Glu Lys Asn Ile
    130                 135                 140

Lys Glu Phe Leu Gln Ser Phe Val His Ile Val Gln Met Phe Ile Asn
145                 150                 155                 160

Thr Ser

(2) INFORMATION FOR SEQ ID NO:6:

(i) SEQUENCE CHARACTERISTICS:
(A) LENGTH: 114 amino acids
(B) TYPE: amino acid
(D) TOPOLOGY: linear

(ii) MOLECULE TYPE: protein

(iii) HYPOTHETICAL: NO

(xi) SEQUENCE DESCRIPTION: SEQ ID NO:6:

Asn Trp Val Asn Val Ile Ser Asp Leu Lys Lys Ile Glu Asp Leu Ile
1               5                   10                  15

Gln Ser Met His Ile Asp Ala Thr Leu Tyr Thr Glu Ser Asp Val His
            20                  25                  30

Pro Ser Cys Lys Val Thr Ala Met Lys Cys Phe Leu Leu Glu Leu Gln
    35                  40                  45

Val Ile Ser Leu Glu Ser Gly Asp Ala Ser Ile His Asp Thr Val Glu
    50                  55                  60

Asn Leu Ile Ile Leu Ala Asn Asn Ser Leu Ser Ser Asn Gly Asn Val
65                  70                  75                  80

Thr Glu Ser Gly Cys Lys Glu Cys Glu Glu Leu Glu Glu Lys Asn Ile
                85                  90                  95

Lys Glu Phe Leu Gln Ser Phe Val His Ile Val Gln Met Phe Ile Asn
            100                 105                 110

Thr Ser

(2) INFORMATION FOR SEQ ID NO:7:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iv) ANTI-SENSE: NO

(xi) SEQUENCE DESCRIPTION: SEQ ID NO:7:

TCGACTGGAA CGAGACGACC TGCT                                        24

(2) INFORMATION FOR SEQ ID NO:8:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iv) ANTI-SENSE: NO

(xi) SEQUENCE DESCRIPTION: SEQ ID NO:8:

AGCAGGTCGT CTCGTTCCAG 20

(2) INFORMATION FOR SEQ ID NO:9:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iv) ANTI-SENSE: NO

(xi) SEQUENCE DESCRIPTION: SEQ ID NO:9:

AAYTGGGTNA AYGTNATH 18

(2) INFORMATION FOR SEQ ID NO:10:

(i) SEQUENCE CHARACTERISTICS:
(A) LENGTH: 17 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iv) ANTI-SENSE: YES

(xi) SEQUENCE DESCRIPTION: SEQ ID NO:10:

ACRTCNGAYT CNGTRTA 17

(2) INFORMATION FOR SEQ ID NO:11:

(i) SEQUENCE CHARACTERISTICS:
(A) LENGTH: 17 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iv) ANTI-SENSE: YES

(xi) SEQUENCE DESCRIPTION: SEQ ID NO:11:

ACRTCRCTYT CNGTRTA 17

(2) INFORMATION FOR SEQ ID NO:12:

(i) SEQUENCE CHARACTERISTICS:
(A) LENGTH: 114 amino acids
(B) TYPE: amino acid
(D) TOPOLOGY: linear

(ii) MOLECULE TYPE: protein

(iii) HYPOTHETICAL: NO

(xi) SEQUENCE DESCRIPTION: SEQ ID NO:12:

Asn Trp Val Asn Val Ile Ser Asp Leu Lys Lys Ile Glu Asp Leu Ile
1               5                   10                  15

Gln Ser Met His Ile Asp Ala Thr Leu Tyr Thr Glu Ser Asp Val His
            20                  25                  30

Pro Ser Cys Lys Val Thr Ala Met Lys Cys Phe Leu Leu Glu Leu Gln
        35                  40                  45

Val Ile Ser Xaa Glu Ser Gly Asp Xaa Xaa Ile His Asp Thr Val Glu
    50                  55                  60

Asn Leu Ile Ile Leu Ala Asn Asn Xaa Leu Ser Ser Asn Gly Asn Xaa
65                  70                  75                  80

Thr Glu Ser Gly Cys Lys Glu Cys Glu Glu Leu Glu Glu Lys Asn Ile
                85                  90                  95

Lys Glu Phe Leu Gln Ser Phe Val His Ile Val Gln Met Phe Ile Asn
            100                 105                 110

Thr Ser